# EUROPEAN PATENT SPECIFICATION

(11) **EP 2 601 305 B1**
(45) Date of publication and mention of the grant of the patent: **08.03.2017**
(21) Application number: 10747078.3
(22) Date of filing: 06.08.2010
(51) Int. Cl.: C12Q 1/68

(54) **A SEQUENCE SPECIFIC FOR FLAVESCENCE DOREE (FD) PHYTOPLASMA, USES THEREOF AND FD DIAGNOSTIC KITS**
FÜR GOLDGELBE VERGILBUNG (FLAVESCENCE DOREE, FD) VERURSACHENDES PHYTOPLASMA SPEZIFISCHE SEQUENZ, IHRE VERWENDUNG UND DIAGNOSEKIT
SÉQUENCE SPÉCIFIQUE POUR PHYTOPLASMA CAUSANT LA FLAVESCENCE DORÉE (FD), UTILISATIONS DE CELLE-CI ET KITS DE DIAGNOSTIC DE FD

(43) Date of publication of application: 12.06.2013
(73) Proprietor: International Plant Analysis and Diagnostics S.R.L., 26900 Lodi (IT)
(72) Inventor: BIANCO, Piero Attilio, I-20052 Monza MB (IT); CASATI, Paola, I-20059 Vimercate MB (IT); DURANTE, Giuseppe, I-20010 Arluno MI (IT)
(74) Representative: Predazzi, Valentina
(86) International application number: PCT/IB2010/053563
(87) International publication number: WO 2012/017271

(56) References cited:
- KR-A- 20100 064 970
- GALETTO L ET AL: "Universal and group-specific real-time PCR diagnosis of flavescence doree (16Sr-V), bois noir (16Sr-XII) and apple proliferation (16Sr-X) phytoplasmas from field-collected plant hosts and insect vectors", ANNALS OF APPLIED BIOLOGY, vol. 147, no. 2, 2005, pages 191-201, XP002609119, ISSN: 0003-4746 cited in the application
- QUAGLINO F ET AL: "Distinct rpsC single nucleotide polymorphism lineages of Flavescence Dor,e subgroup 16SrV-D phytoplasma co-infect Vitis vinifera L.", FOLIA MICROBIOLOGICA, vol. 55, no. 3, May 2010 (2010-05), pages 251-257, XP002609120, ISSN: 0015-5632
- MARGARIA P ET AL: "Detection of Flavescence doree phytoplasma in grapevine by reverse-transcription PCR", PLANT DISEASE, vol. 91, no. 11, November 2007 (2007-11), pages 1496-1501, XP002609121, ISSN: 0191-2917
- DUDUK B ET AL: "Identification of phytoplasmas associated with grapevine yellows in serbia", JOURNAL OF PHYTOPATHOLOGY (BERLIN), vol. 152, no. 10, October 2004 (2004-10), pages 575-579, XP002609122, ISSN: 0931-1785
- HREN M ET AL: "Real-time PCR detection systems for Flavescence doree and Bois noir phytoplasmas in grapevine: comparison with conventional PCR detection and application in diagnostics", PLANT PATHOLOGY (OXFORD), vol. 56, no. 5, October 2007 (2007-10), pages 785-796, XP002609123, ISSN: 0032-0862 cited in the application
- MARTINI M ET AL: "Genetic variability among flavescence doree phytoplasmas from different origins in Italy and France", MOLECULAR AND CELLULAR PROBES, ACADEMIC PRESS, LONDON, GB LNKD- DOI:10.1006/MCPR.2002.0410, vol. 16, no. 3, 1 June 2002 (2002-06-01), pages 197-208, XP004470998, ISSN: 0890-8508 cited in the application
- ARNAUD GUILLAUME ET AL: "Multilocus sequence typing confirms the close genetic interrelatedness of three distinct flavescence doree phytoplasma strain clusters and group 16SrV phytoplasmas infecting grapevine and alder in Europe", APPLIED AND ENVIRONMENTAL MICROBIOLOGY, vol. 73, no. 12, June 2007 (2007-06), pages 4001-4010, XP002609124, ISSN: 0099-2240
- FILIPPIN L ET AL: "Molecular characteristics of phytoplasmas associated with Flavescence doree in clematis and grapevine and preliminary results on the role of Dictyophara europaea as a vector", PLANT PATHOLOGY (OXFORD), vol. 58, no. 5, October 2009 (2009-10), pages 826-837, XP002609125, ISSN: 0032-0862
- DAVIS ROBERT E ET AL: "Revised subgroup classification of group 16SrV phytoplasmas and placement of flavescence doree-associated phytoplasmas in two distinct subgroups", PLANT DISEASE, vol. 85, no. 7, July 2001 (2001-07), pages 790-797, XP002609126, ISSN: 0191-2917
- EPPO BULLETIN, vol. 37, no. 3, December 2007 (2007-12), pages 536-542, XP002609127, ISSN: 1365-2338, DOI: 10.1111/j.1365-2338.2007.01161.x
- DATABASE MEDLINE [Online] US NATIONAL LIBRARY OF MEDICINE (NLM), BETHESDA, MD, US; 5 July 2008 (2008-07-05), XP002609128, Database accession no. CU469464
- BULGARI DANIELA ET AL: "Endophytic Bacterial Diversity in Grapevine (Vitis vinifera L.) Leaves Described by 16S rRNA Gene Sequence Analysis and Length Heterogeneity-PCR", JOURNAL OF MICROBIOLOGY, vol. 47, no. 4, August 2009 (2009-08), pages 393-401, XP002609129, ISSN: 1225-8873
- FIRRAO G ET AL: "'Candidcatus Phytoplasma', a taxon for the wall-less, non-helical prokaryotes that colonize plant phloem and insects", INTERNATIONAL JOURNAL OF SYSTEMATIC AND EVOLUTIONARY MICROBIOLOGY, vol. 54, no. Part 4, July 2004 (2004-07), pages 1243-1255, XP002609130, ISSN: 1466-5026 cited in the application
- LEE ING-MING ET AL: "Phytoplasma: Phytopathogenic mollicutes", ANNUAL REVIEW OF MICROBIOLOGY ANNUAL REVIEWS {A}, 4139 EL CAMINO WAY, PALO ALTO, CA, 94303-0139, USA SERIES : ANNUAL REVIEW OF MICROBIOLOGY (ISSN 0066-4227), 2000, pages 221-255, XP002609131, cited in the application

### SEQUENCE LISTING

<110> Unniversità degli studi di Milano
<120> A SEQUENCE SPECIFIC FOR FLAVESCENCE DOREE PHYTOPLASMA (FD), USES THEREOF AND FD DIAGNOSTIC KITS
<130> BW569R
<160> 6
<170> PatentIn version 3.3
<210> 1
   <211> 381
   <212> DNA
   <213> Phytoplasma sp.
<220>
   <221> allele
   <222> (108)..(108)
   <223> when nucleotide 107 is C the sequence is specific for the phytoplasmas sp. associated with vine Flavenscence Dorée
<220>
   <221> allele
   <222> (171)..(171)
   <223> when nucleotide 171 is G the sequence is specific for the phytoplasmas sp. associated with vine Flavenscence Dorée
<400> 1
   atgattcaag taaatacccg tttaaaagta gctgataata gtggtgctaa agaagtttta     60
   gttattaata ttcctggttc aagtaaaaga cgttatgctc aaataggcga tgttgttgta    120
   gtcgctatta aaaaaagtat tccatctgga atggtaaaat caggagaaaa gtctaaagct    180
   ttaattttac gtactaaaaa aggcttagca ggaaaaaatg gttcttatat taaatttgat    240
   gataacgcag tagttattat caaagatgat atgactttaa aagggacaag gatttttgga    300
   cctgtagtta aaaatcaaaa actaaaagat agtaaatttt ctaaatttct ttctttagct    360
   gaaaaagttt ttgtaatatg a                                              381
<210> 2
   <211> 26
   <212> DNA
   <213> Phytoplasma sp.
<400> 2
   tcctggttca agtaaaagac gttatg 26
<210> 3
   <211> 17
   <212> DNA
   <213> Phytoplasma sp.
<220>
   <221> misc_feature
   <222> (1)..(1)
<220>
   <221> misc_feature
   <222> (297)..(297)
   <223> n is C or T
<400> 5
<210> 6
   <211> 126
   <212> PRT
   <213> Phytoplasma sp.
<220>
   <221> misc_feature
   <222> (4)..(4)
   <223> The 'Xaa' at location 4 stands for Ile, Val, or Leu.
<220>
   <221> misc_feature
   <222> (6)..(6)
   <223> The 'Xaa' at location 6 stands for Lys, Asn, Arg, Ser, Thr, Ile, or Met.
<220>
   <221> misc_feature
   <222> (57)..(57)
   <223> The 'Xaa' at location 57 stands for Lys, or Asn.
<220>
   <221> misc_feature
   <222> (82)..(82)
   <223> The 'Xaa' at location 82 stands for Lys, or Asn.
<220>
   <221> misc_feature
   <222> (87)..(87)
   <223> The 'Xaa' at location 87 stands for Ile, or Met.
<220>
   <221> misc_feature
   <222> (99)..(99)
   <223> The 'Xaa' at location 99 stands for Leu, or Phe.
<400> 6
   Met Ile Gln Xaa Asn Xaa Arg Leu Lys Val Ala Asp Asn Ser Gly Ala
   1               5                   10                  15
   Lys Glu Val Leu Val Ile Asn Ile Pro Gly Ser Ser Lys Arg Arg Tyr
               20                  25                  30
   Ala Gln Ile Gly Asp Val Val Val Val Ala Ile Lys Lys Ser Ile Pro
           35                  40                  45
   Ser Gly Met Val Lys Ser Gly Glu Xaa Ser Lys Ala Leu Ile Leu Arg
       50                  55                  60
   Thr Lys Lys Gly Leu Ala Gly Lys Asn Gly Ser Tyr Ile Lys Phe Asp
   65                  70                  75                  80
   Asp Xaa Ala Val Val Ile Xaa Lys Asp Asp Met Thr Leu Lys Gly Thr
                   85                  90                  95
   Arg Ile Xaa Gly Pro Val Val Lys Asn Gln Lys Leu Lys Asp Ser Lys
               100                 105                 110
   Phe Ser Lys Phe Leu Ser Leu Ala Glu Lys Val Phe Val Ile
           115                 120                 125
<210> 7
   <211> 126
   <212> PRT
   <213> Phytoplasma sp. FD associated strain
<400> 2

## Description

The present invention was granted a foreign filing license issued by the Ministero Dello Sviluppo Economico, U.I.B.M. Divisione XI, on the 26th of May 2010, Protocol number 59395.

The present invention relates to a new genomic sequence specific for the phytoplasma causing Flavescence dorée (FD) in vines, to a method for the diagnosis of FD in vines and parts thereof including rootstocks and to a kit for the detection of the phytoplasma agents of FD from plant samples.

### STATE OF THE ART

Grapevine yellows (GY), a complex of diseases that were originally thought to be caused by viruses, are now known to have a phytoplasma aetiology. The first GY disease described for *Vitis vinifera*, and the most widely known among GY diseases, is undoubtedly Flavescence dorée (FD), which was reported for the first time in south-west France in the 1950's (Caudwell, 1957), and then spread to other viticultural districts of France, northern Italy and neighbouring European countries, including Spain, Portugal and Serbia.
Bois noir (BN), whose symptoms are indistinguishable from those of FD, was also first reported from France, and then from the most important viticultural areas of Europe.
Almost identical symptoms of the GY syndrome are caused by different phytoplasmas and appear on leaves, shoots and clusters of grapevine. Typical symptoms include discoloration and necrosis of leaf veins and leaf blades, downward curling of leaves, lack or incomplete lignifications of shoots, stunting and necrosis of shoots, abortion of inflorescences and shrivelling of berries. Those symptoms are related to callose deposition at the sieve plates and subsequent degeneration of the phloem. Although no resistant cultivars of *Vitis vinifera* or rootstocks are known so far, the various grape varieties differ considerably as far as symptom severity is concerned. It ranges from fast decline and death in highly susceptible cultivars to tolerant rootstocks as symptomless carriers of the pathogen.

Phytoplasmas are microscopic plant pathogens, similar to, but much smaller than bacteria. They are phloem-limited, non-helical and wall-less prokaryotes (Firrao, G., et al, 2004.). Differently form viruses, phytoplasmas have their own metabolism, a highly reduced metabolism, and some of the molecules essential for their survival are acquired from the host. They represent a monophyletic clade within the class Mollicutes, which is currently divided into at least 15 subgroups on the basis of sequence analyses of various conserved genes. A new taxon, '*Candidatus* Phytoplasma' has been established and various groups or subgroups have been described recently as '*Ca*. Phytoplasma' species.
The full genomes of four phytoplasmas are sequenced, providing new information on their biology and their interaction with plant and insect hosts ("Ca. Phytoplasma asteris" strain AY-WB NC_007716 and OY NC_005303 "Ca. P. mali" C_011047, "*Ca*. P. australiense" N_010544).
The available data confirm their lack of genes for autonomous metabolism and their reliance on intracellular parasitism. Phytoplasmas of several groups have been found to be consistently associated with GY. They propagate through a vectoring insect; also the propagation of infected grapevines made by man contributes to GY spreading over long distance. This implies a high risk of dissemination of GY to regions where new outbreaks could be provoked if competent vectors existed or would be introduced, too.
GY, however, have different phytoplasma species as causal agent, as well as different insect vectors, which are either leafhoppers or plant-hoppers (Homoptera: Auchenorrhyncha) that feed specifically or just occasionally on the vines. It is worth noting that two or more different phytoplasma species may infect simultaneously individual grapevines, thus causing mixed infections.
Although phytoplasmas are non culturable micro-organisms and, in the case of GY, Koch's postulates have not yet been fulfilled, when phytoplasmas of a specific group or subgroup are found consistently associated with a specific grape disease, they are regarded as being its causal agents. In fact, the identification of phytoplasmas as the cause of GY was made possible over the last decades by molecular detection methods, which allow to distinguish one from another the phytoplasma species involved in any single disease etiolology.

The classification of phytoplasmas, which are uncultivable and currently described under the provisional genus "*Candidatus* Phytoplasma," is mainly based on 16S rRNA gene phylogeny, genomic diversity, and plant and insect host ranges. Phytoplasmas are associated with grapevine yellows.

In 2003 Boudon-Padieu (Boudon-Padieu, E., 2003) listed several phytoplasmas that were found to be consistently associated with GY. Stolbur (16SrXII-A) phytoplasma, the causal agent of Bois noir (BN), was reported from grapevine in Chile and phytoplasmas of the same group, but also of 16SrI (aster yellows) group were identified in cultivar Syrah in France. BN has now been reported also in Canada. Stolbur phytoplasma was also identified in GY diseased vines in Ukraine, and its presence in Serbia could be confirmed. Recently, new and repeated outbreaks of BN caused problems in various viticultural regions in Europe. These data confirm that BN is an endemic disease in Europe, Asia Minor and the Mediterranean. Aster yellows (16SrI-B), clover phyllody (16SrI-C) and elm yellows (16Sr-V) phytoplasmas were found sporadically in GY diseased grapevines in Italy (18), and infection of grapevine by aster yellows (16SrI-B) phytoplasma was reported from Tunisia. Infection of papaya and grapevine by "*Ca*. Phytoplasma australiense" was reported from Israel, however, confusion with stolbur phytoplasma, which is also present in this region, is possible. A survey in Italy revealed the presence of GY in 80% of the inspected vineyards. BN is widespread all over the country whereas Flavescence dorée (FD) is mainly restricted to the northern regions. Recently, the presence of vines infected by FD/C phytoplasmas has been reported in Slovenia and Serbia. The FD phytoplasma belongs to the 16SrV taxonomic group (Lee et al., 2000) and it is the most important and destructive phytoplasma disease of grapevines and its causing phytoplasmas show a high vector specificity, being transmitted under natural conditions only by the leafhopper S. titanus (Hemiptera: Cicadellidae). S. titanus spends its whole life cycle on grapevines. It is a highly mobile and efficient vector that is largely responsible for the epidemic spread of FD. Both nymphs and adults are able to acquire the phytoplasma while feeding. After a latent period they are able to transmit the disease until they die.

FD has been called "catastrophic" in France, and it is so severe that in the EU it is subject to quarantine.

Vines affected by the disease are characterised by colour alterations of the leaves (yellowing or reddening), low shoots lignifications and bunches dryness. Impacts include reduced vitality of vines, yield reductions, and reduced wine quality due to high acid and low sugar contents of fruit from infected plants. No symptoms or very faint symptoms are visible on the most popular rootstock. This fact plays a critical role in the FD spreading, and only extended and continuous bio molecular controls may help in keeping the mother vine plant free from the phytoplasmas. When no control measures are used, in the presence of the insect vectors, the disease spreads rapidly, affecting up to 80-100% of vines within a few years. FD has destroyed large viticultural areas in France and Italy, and it is spreading all over southern Europe despite mandatory control programs.

Popular cultivars such as Chardonnay, Cabernet Sauvignon, Pinot noir, Riesling, Sauvignon blanc, and Sémillon are highly susceptible to FD. Some cultivars such as Sangiovese and Garganega are extremely susceptible, and, if frequently inoculated, the plants are strongly damaged; sometimes the diseased vines cannot survive to winter's temperature, and die.

FD is caused by two phytoplasma strains: the first belongs to the 16SrVD taxonomic group, and the second belongs to the 16SrVC taxonomic group.

Reliable detection of phytoplasmas in grapevine is still a challenge although a wide variety of diagnostic tools is available. Low and yearly as well as seasonally fluctuating titres of the pathogens and their irregular distribution in infected vines cause problems. Visual inspection is impeded by latent infection in some rootstocks and long periods of symptomless infection in *Vitis vinifera.*

As from 1982, monoclonal antibodies and polyclonal antisera were produced for the detection of FD phytoplasmas through immunization of rabbits with partially purified extracts from FD-infected *Vicia faba* plants or from the experimental vector E. variegatus. These antisera were also used for observing phytoplasmas by means of immunosorbent electron microscopy (ISEM) and fluorescence light microscopy. Polyclonal antisera were reported as successfully employed for dot blot and ELISA-based diagnosis of the FD phytoplasma in infected broad bean plants and in single S. titanus individuals collected in GY-affected vineyards. However, these antisera did not yield acceptable results for FD agent detection in grapevines in term of sensitivity and specificity. In summary, no reliable serological tool is so far available for E.L.I.S.A. tests, or similar methods, in grapevine tissues.

Actually, several methods for FD detection have been developed; they include conventional PCR/RFLP methods based on the analysis of ribosomal and non-ribosomal genes (Lee et al., 2004 ; Botti et al., 2007) and more recently real-time PCR assays (RT-PCR) (Bianco et al. 2004; Galetto et al. 2005 ; Angelini et al. 2007; Hren et al., 2007). Nevertheless, none of these assays allows to easily and readily distinguish FD phytoplasma from other phytoplasmas sporadically found in grapevine (EY= elm yellow, ALY=alder yellow), as very long times for sample processing are required (PCR/RFLP).

Several nucleotide sequences related to "*Ca.* Phytoplasma vitis" are available in online databases (16S rpl22, rps3, secY etc.) but none of them is suitable for the design of a RT PCR assay for the specific identification of FD associated phytoplasma. The only method currently available to uniquely identify FD consists of a molecular diagnostic assay comprising two main phases with several analytical steps (Bianco et al., 2004). The main phases can be summarised as follows: the DNA extracted from grapevine samples is subject to a first gene amplification (PCR end-point) producing amplicons that are subsequently used for the second main step as template for a RT PCR assay. In detail, two different primer sets, as reported by Lee et al., 1993 or by Deng and Hiruki, 1991; Schneider et al., 1995, designed for the amplification of the 16Sr DNA from all known phytoplasmas, are used for a first round of amplification. The amplicons are then subject to a nested PCR using primers designed for the specific amplification of DNA from aster yellows and stolbur group (Davis et al., 1997), or primers designed for the specific amplification of the elm yellows group (Lee et al., 1994).

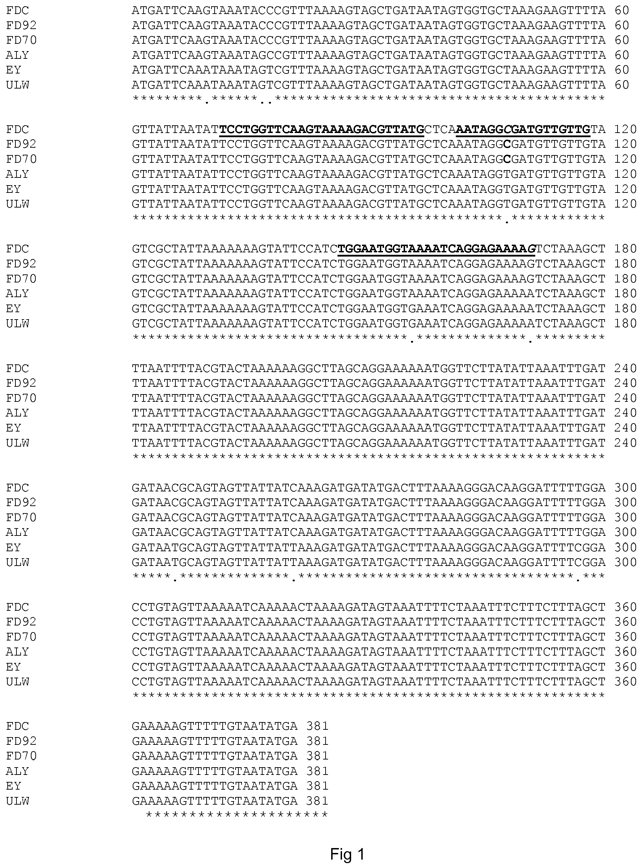

In order to develop a RT PCR assay for simultaneous detection of phytoplasmas belonging to the 16SrV-C and 16SrV-D subgroups, specific primers were designed. The amplified fragment contains the *Bfa*I cleavage site that is present in both the 16SrV-C and 16SrV-D subgroups. In the same fragment a single base mutation (T instead of C) occurs at position 1068 of EY1 and ULW reference strains thus disrupting the *Bfa*I restriction site (Fig. 1). The presence of this restriction site has been suggested as a distinctive marker for the identification of phytoplasmas related to the FD agents (Lee et al., 1998) and was recently adopted for identification of phytoplasmas associated with FD in Italy (Pasquini et al., 2001).

The disadvantage of this methodology, which includes two amplification reactions, consists of long execution time and high cost. In addition, the skilled person is well aware that several analytical steps can involve risks of cross-contamination of samples. Given the "catastrophic" effect caused by FD, the symptomless rootstocks, the difficulty in distinguishing FD from EY and or ALY, and the valuable cultivars that are severely affected by this disease, it is evident that a fast and reliable method for the detection of the disease could enable the activation of national and international control programs, and could favour preventative measures against the spreading of the disease in plant nurseries, green centres, and breeding grounds.

No curative tools or chemical treatment are so far effective against the pathogen. For this reason, following the new and destructive FD outbreaks registered in the 1990's in France, then in northern Italy and now in the balcanian areas, and since FD is included in the EPPO quarantine list, several initiatives (i.e. the Italian Ministry of Agriculture enacted a decree for the compulsory control of the disease, D.M. n. 32442 of May 31^{st}, 2000: "Misure per la lotta obbligatoria contro la Flavescenza Dorata della vite", published in the G.U. n.159 of July 10th, 2000) established officially the measures that had to be enforced to contain FD infections. In detail, in those areas where FD is present as focus, the eradication of the disease by elimination and destruction of the infected plant is compulsory. In addition, also insecticide treatments twice a year against *Scaphoideus titanus* Ball are mandatory. Further control measures can be imposed by local Plant Protection Services (PPS), according to each specific situation, including the complete eradication of vineyards with symptomatic plants.

Phytoplasmas associated with German Palatinate grapevine yellows phytoplasmas (PGY) have been reported only once in literature and no other cases of this infection have been reported ever since in grapevine. They seem to be transmitted by the alder leafhopper Oncopsis alni (Schrank) (Maixner, M., and W. Reinert. 1999, . Maixner, M., W. Reinert, and H. Darimont. 2000), and they were classified as members of the group 16SrV on the basis of their 16S rRNA gene. Palatinate Grapevine Yellows (PGY) phytoplasmas, usually occurring in non-grapevine plants, were also found sporadically in grapevine. PGY emerged as a new grapevine yellows disease in the 1990s in Germany. In vineyard, the vector may inoculate grapevine by erratic feeding on this atypical host. Corresponding to the low risk of infection, PGY is almost exclusively found in rather old vineyards and with a low incidence (<1%), but it seems to occur wherever grapevine is grown in close association with alder trees (Maixner et al., 2000). Until now, experimental transmission of PGY phytoplasmas by *S*. *titanus* specimens has remained unsuccessful (M. Maixner and E. Boudon-Padieu, unpublished results).

It is important to look at the epidemiology of diseases caused by phytoplasma belonging to 16SrV. While FD phytoplasma is strongly epidemic, and it is recognized by the European and Mediterranean Plant Protection Organization (EPPO) as a quarantine pest (EPPO/CABI, 2003), other phytoplasmas of the same subgroup, like EY, PGY and ALY, are not epidemic. They are sporadically found in grapevine, probably because their vectors feed on grapevine occasionally and they do not survive enough to acquire the pathogen from the grapevine. The grapevine growers, thus, are forced to eliminate the plants infected by FD phytoplasmas, whereas it is neither mandatory, nor necessary to destroy the plant infected by EY, ALY and other occasionally phytoplasmas found so far in grapevine.

Hence, due to the severe treatments that follow an FD infection (or a suspected FD infection) such as eradication of the plants, insecticide treatments, quarantine and others, and due to the fact that when an FD infection is, indeed, present no rootstock can be saved and no propagation of the vine is possible, whereas, by way of example, when an EY or ALY infection is present the same measures need not to be adopted, it is evident that a diagnostic test and/or tool allowing an unambiguous and specific identification of the presence or of the absence of FD in a phytoplasmatic infection would be very useful and could avoid drastic measures and unnecessary losses of precious vine plants.

### DESCRIPTION

The present description provides a new nucleotide sequence of SEQ ID NO 5 coding for the gene rpl14 (ribosomal protein 114 gene) of phytoplasmas belonging to the 16SrV-C and 16SrV-D subgroups wherein the group (16SrV) comprises at least the infecting phytoplasmas associated with other diseases such as Alder yellow (ALY), Elm Yellow (EY), Elm witch's broom (ULW) and FD, sometimes found in asymptomatic grapevines. (also named "Ca. phytoplasma vitis" although the nomenclature for phytoplasmas is still under discussion in the scientific world).
The rpl14 gene codes for the L14 ribosomal protein that is believed to bind the 23S rRNA between the centers for peptidyl transferase and GTPase. In detail, the ribosomal peptidyl transferase center (PTC) resides in the large ribosomal subunit and catalyzes the two principal chemical reactions of protein synthesis: peptide bond formation and peptide release. The peptide release should more strongly depend on chemical catalysis likely involving an rRNA group of the PTC. (Polacek, N. and Mankin, A.S, 2005).

The newly provided sequence allows for the recognition of different phytoplasmas belonging to said subgroups and provides a new tool for a subgroup-specific identification of these microorganisms.

The sequence is disclosed as SEQ ID NO 5 and the protein coded by the sequence is disclosed in SEQ ID NO 6.
SEQ ID NO 5 is almost identical for FD, ALY, EY and ULW, nevertheless, certain nucleotides defined as "n" in the sequence listing and the related amino acids defined as "Xaa" in the sequence listing allow the distinction between the phytoplasma inducing FD in the vine and the other three known phytoplasmas belonging to the same subgroups of FD, namely ALY, EY and ULW.

The present sequence hence provides a useful tool for the specific detection of the presence of the FD related phytoplasma in a vine sample. The sequence of the rpl14 gene (381 bp) specific for the FD related phytoplasma is depicted in SEQ ID NO 1, and codes for the ribosomal protein rpL14 specific for the phytoplasma associated with FD that differs for two single-nucleotide polymorphisms (SNPs) from other phytoplasmas belonging to the taxonomic group 16SrV.

This sequence is workable as a diagnostic tool as it comprises two SNPs specific for FD in position 108 and in position 171. Due to the SNPs identified by the authors of the present invention, it is now possible, reading the teachings of the present description, to design a diagnostic assay that specifically identifies the phytoplasma associated with FD.

As said herein above, the rpl14 gene is characterized by two SNPs in position 108 and 171 of SEQ ID NO 1 that allow to distinguish phytoplasma strains associated with FD from other phytoplasmas (not associated with FD) such as the phytoplasmas associated with EY and ALY, occasionally present in grapevines. The present description hence provides a nucleotide sequence of SEQ ID NO 5 or the sequence complementary to SEQ ID NO 5, a nucleotide sequence of SEQ ID No. 1 or the sequence complementary to SEQ ID NO 1 or fragments thereof, said fragments comprising one or both of nucleotides 108 and 171 of SEQ ID NO 1, or of their complementary nucleotides, or said fragments being suitable for the amplification of a fragment of SEQ ID NO 1, or the sequence complementary to SEQ ID NO 1 comprising one or both of nucleotides 108 and 171 of SEQ ID NO 1, or of their complementary nucleotides, wherein said sequence or fragments thereof are specific for the plant phytoplasma strains associated with the vine disease Flavescence dorée, a nucleotide vector comprising a nucleotide sequence of SEQ ID NO 5 or the sequence complementary to SEQ ID NO 5, a nucleotide sequence of SEQ ID NO 1, or the sequence complementary to SEQ ID NO 1, or fragments thereof, said fragments comprising one or both of nucleotides 108 and 171 of SEQ ID NO 1, or of their complementary nucleotides, a method for the diagnosis of the vine disease Flavescence dorée comprising the step of
a) identifying one or both of the nucleotides in position 108 and 171 of SEQ ID NO 5, or of their complementary nucleotides in the sequence complementary to SEQ ID NO 1, in a plant sample comprising phloem wherein the presence of a C residue in position 108 of SEQ ID NO 5, or of a G residue in its complementary nucleotide position in the sequence complementary to SEQ ID NO 5, and/or of a G residue in position 171 of SEQ ID NO 1, or of a C residue in its complementary nucleotide position in the sequence complementary to SEQ ID NO 1, indicates the presence of at least one of the phytoplasma strains associated with the vine disease Flavescence dorée in the sample, and a kit for the diagnosis of the vine disease Flavescence dorée comprising reagents for the identification one or both of the nucleotides in position 108 and 171 of SEQ ID NO 5, and or of their complementary nucleotides in the sequence complementary to SEQ ID NO 5 in a plant phloem.

### DETAILED DESCRIPTION OF THE FIGURES

Figure 1 represents an alignment of the rpl14 gene of three strains of FD and ALY, EY and ULW (the last being a phytoplasma strain associated with elm witch's broom identified in elm trees. It belongs to the taxonomic group 16SrV-A, now named "Ca. Phytoplasma ulmi"). The alignment clearly demonstrates a SNP C/T in position 108 and a SNP G/A in position 171 each allowing the specific identification of phytoplasmas associated with FD.

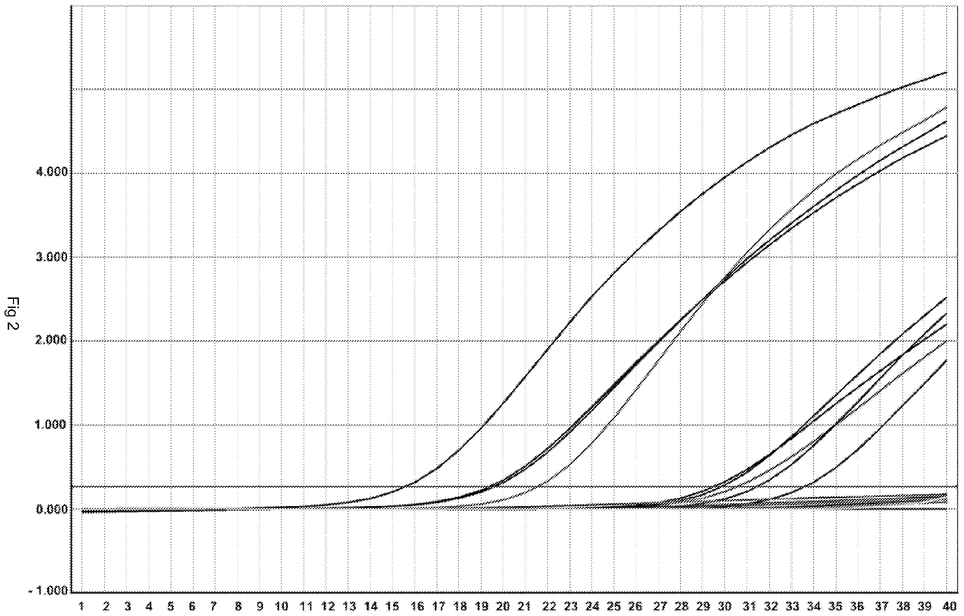

Figure 2 shows an amplification plot example. The Cycle number is shown along the X-axis and arbitrary fluorescence units (actually these are fold increase over background fluorescence) are shown on the Y-axis. It is possible to observe an amplification signal only for the grapevine sample infected by FD phytoplasma as well as for the positive control. No amplification was detected in negative control. This shows that FD RT PCR assay was specific to FD phytoplasma.

### DETAILED DESCRIPTION OF THE SEQUENCES

SEQ ID No. 1: FD associated phytoplasma's rpl14 gene Nucleotides no 108 and 171 underlined and in bold corresponds to the SNP of interest.
   Underlined, from nucleotide 72 to 97 the forward primer of SEQ ID NO 2 below
SEQ ID No 2:
   tcctggttcaagtaaaagacgttatg
      from nucleotide 102 to 119 the nested primer of SEQ ID NO 3 beolw
SEQ ID No 3
   aataggcgatgttgttg
   and from nucleotide 147 to 171 the sequence complementary to the reverse primer of SEQ ID No 4 below:
SEQ ID No 4:
   cttttctcctgattttaccattcca
SEQ ID No 5: Subgroups 16SrV-C and 16SrV-D phytoplasma's rpl14 gene wherein n in position 10 is G or A ,n in position 17 is C or G, n in position 18 is C or T,n in position 108 is C or T, n in position 156 is A or G, n in position 171 is G or A, n in position 246 is C or T, n in position 261 is C or T, n in position 297 is C or T SEQ ID No 6: Subgroups 16SrV-C and 16SrV-D phytoplasma's L14 ribosomal protein wherein 'Xaa' at location 4 stands for Ile, Val, or Leu., 'Xaa' at location 6 stands for Lys, Asn, Arg, Ser, Thr, Ile, or Met, 'Xaa' at location 57 stands for Lys, or Asn, 'Xaa' at location 82 stands for Lys, or Asn, 'Xaa' at location 87 stands for Ile, or Met, 'Xaa' at location 99 stands for Leu, or Phe.
SEQ ID No 7: FD associated strain L14 ribosomal protein

### DETAILED DESCRIPTION OF THE INVENTION

Objects of the invention are:
1. A method for the diagnosis of the vine disease Flavescence dorée comprising the step of
   a) identifying one or both of the nucleotides in position 108 and 171 of SEQ ID NO 5, and/or their complementary nucleotides in the sequence complementary to SEQ ID NO 5, in a plant sample comprising phloem;
   wherein the presence of a C residue in position 108 of SEQ ID NO 5, and/or of a G residue in its complementary nucleotide in the sequence complementary to SEQ ID NO 5 and/or of a G residue in position 171 of SEQ ID NO 5, and/or of a C residue in its complementary nucleotide, indicates the presence of at least one of the phytoplasma strains associated with the vine disease Flavescence dorée in the sample.
2. The method of point 1 wherein the identification of step a) comprises a first step wherein a PCR, real-time PCR, Ligation, Allele Specific Hybridization, primer extension, invasive cleavage or sequencing reaction is carried out, and a second step wherein the product obtained in the first step is detected by monitoring the light emitted by said product, or measuring the mass of said products, or monitoring the radioactivity emitted by said product, or by sequencing said product.
3. The method of any one of points 1 or 2 wherein said monitoring of the light emission is carried out by monitoring fluorescence, luminescence, time resolved fluorescence, fluorescence resonance energy transfer, or fluorescence polarisation, said measuring of the mass is carried out by mass spectrometry or heteroduplex analysis, and said monitoring of the radioactivity is carried out with radioactivity sensitive tools.
4. The method of any one of points 1-3 wherein said method is performed on solid phase format such as microarray or microplate.
5. The method of any one of points 1-3 wherein said identification is carried out with a RT PCR using a forward primer of SEQ ID NO 2, an allele specific probe of SEQ ID NO 3 and a reverse primer of SEQ ID NO 4.
6. A kit for the diagnosis of the vine disease Flavescence dorée comprising reagents for the identification of the nucleotides in position 108 and 171 of SEQ ID NO 5 or 1 and/or of their complementary nucleotides in the sequence complementary to SEQ ID NO 5 or 1 in a vine plant phloem.
7. The kit of claim 6 further comprising solid supports wherein oligonucleotides for the identification of one or both of the nucleotides in position 108 and 171 of SEQ ID NO 5 or 1 and/or one or both of their of its complementary nucleotide in the sequence complementary to SEQ ID NO 5 or 1 are anchored in known positions on said support in one or more copy per position.
8. The kit of point 7 further comprising labelled probes and/or oligonucleotides corresponding and/or complementary to a region upstream and/or downstream of one or both of the nucleotides in position 108 and 171 of SEQ ID NO 5 or 1 and/or overlapping a region comprising one or both of the nucleotides in position 108 and 171 of SEQ ID NO 5 or 1.
9. The kit of point 8 wherein one or more of said probes and/or oligonucleotides is labelled.
10. A nucleotide sequence of SEQ ID NO 1, said sequence coding for the rpl14 gene (381 bp) specific for the FD related phytoplasma.

The present description provides a nucleotide sequence of SEQ ID NO 5 or the sequence complementary to SEQ ID NO 5 coding for the rpl14 gene of phytoplasmas belonging to the 16SrV-C and 16SrV-D subgroups.

The description provides also a nucleotide sequence of SEQ ID NO 5 or the sequence complementary to SEQ ID NO 5 or fragments thereof, said fragments comprising one or both of nucleotides 108 and 171 of SEQ ID NO 5 or of their complementary nucleotides, or said fragments being suitable for amplification of a fragment of SEQ ID NO 5 or the sequence complementary to SEQ ID NO 5 comprising one or both of nucleotides 108 and 171 of SEQ ID NO 5 or of their complementary nucleotides.

The presence of a C in position 108 of SEQ ID NO 5 (or of a G in the corresponding position in the complementary strand) and/or of a G in position 171 of SEQ ID NO 5 (or of a C in the corresponding position of the complementary strand) in SEQ ID NO 5 is specific for the phytoplasma associated with FD vine disease.

The present description also provides an amino acid sequence of SEQ ID NO 6 coding for the L14 ribosomal protein of phytoplasmas belonging to the 16SrV-C and 16SrV-D subgroups, and an amino acid sequence of SEQ ID NO 7 coding for the L14 ribosomal protein of the FD associated phytoplasma.

The present description provides a nucleotide sequence of SEQ ID NO 1 or the sequence complementary to SEQ ID NO 1 or fragments thereof, said fragments comprising one or both of nucleotides 108 and 171 of SEQ ID NO 1 or of their complementary nucleotides, or said fragments being suitable for amplification of a fragment of SEQ ID NO 1 or the sequence complementary to SEQ ID NO 1 comprising one or both of nucleotides 108 and 171 of SEQ ID NO 1 or of their complementary nucleotides, wherein said sequence or fragments thereof are specific for the plant phytoplasma strains associated with the vine disease FD .
The sequence, allowing to specifically detect the plant phytoplasma strains associated with the vine disease FD can hence be SEQ ID NO 1 or the sequence complementary to it, and the fragments can be fragments of SEQ ID NO 1 or fragments of the sequence complementary to it provided that they comprise one or both of nucleotides 108 of SEQ ID NO 1 (a C nucleotide or it's G complement in the case of a fragment of the complementary sequence) and 171 (a G nucleotide or it's C complement in the case of a fragment of the complementary sequence) or that they can be used for amplifying a fragment of SEQ ID NO 1 comprising one or both of said nucleotides, as said particular nucleotides represent SNPs that render SEQ ID NO 1 specific for the plant phytoplasma strains associated with the vine disease Flavescence dorée and allow, each alone or in combination with the other, a distinction of said phytoplasma from highly similar phytoplasmas, belonging to the same taxonomic group, that are not associated with FD.

The fragments should be fragments of a dimension suitable to be used for the detection of the phytoplasma strains associated with the vine disease FD with any common technique known for SNPs detection in the art.

Normally, the skilled person would know the suitable size for the fragments that could be from about 10, 20, 30, 40, 50, 100, 150, 200 nucleotides to the full sequence in case of a complete sequencing of the gene for identification of the FD related phytoplasma.

The fragments of the present description can be used as probes for PCR, as probes for microarray detection of SNPs, or they can be the products of the techniques used for the SNPs identification. Hence, according to the technique used in a method for identification one or both of nucleotides 108 and 171 of SEQ ID NO 5, the fragments can be upstream and/or downstream fragments with respect to nucleotide 108 and/or nucleotide 171 of SEQ ID NO 5, or can be fragments comprising one of the aforementioned nucleotides or both of the aforementioned nucleotides. The skilled person is well aware that, when detection of a SNP is involved, the identification of the nucleotide(s) of interest can be carried on either strand of the nucleotide sequence, hence, the fragments of the invention can be designed either to identify one or both of nucleotides 108 and 171 of SEQ ID NO 5 or 1, and/or to identify one or both of the nucleotides complementary to nucleotides 108 and 171 of the nucleotide sequence complementary to SEQ ID NO 5 or 1 that is implicitly disclosed by the disclosure of SEQ ID NO 5 or 1. The following description will clarify in more detail the fragments of the invention, all of them being characterised by the fact that they can be used for the identification of the aforementioned SNPs.

SEQ ID NO 5 is specific for the group of phytoplasmas comprising the phytoplasmas causing FD, ALY, EY and ULW

On the other hand, the nucleotide sequence of SEQ ID NO 1 or fragments thereof herein disclosed are specific for both strains of the phytoplasma associated with FD known in the art, i.e. FD associated strains belonging to the taxonomic group 16SrVD as well as FD causing strains belonging to the taxonomic group 16SrVC. Hence, SEQ ID NO 1 represents one of the possible embodiments of SEQ ID NO 5, in particular the embodiment wherein nucleotide 108 is C and nucleotide 171 is G. As already indicated in the present specification, the sequence or fragments thereof herein described are applicable as diagnostic tools for the identification, in a grapevine sample, of the phytoplasma associated to the vine disease Flavescence dorée.

The description also provides vectors such as cloning vectors or expression vectors comprising a nucleotide sequence of SEQ ID NO 5 or of SEQ ID NO 1 or fragments thereof, said fragments comprising one or both of nucleotides 108 and 171 of SEQ ID NO 5 or 1.

The vector can be used either as a positive or negative control in a diagnostic method for the detection of FD associated phytoplasma in a vine phloem sample depending on the nucleotides in position 108 and 171 (C and G, respectively, will provide a positive control whether other nucleotides will provide a negative control) or it could also be used as research tool for the development of antibodies and/or for the study of possible therapies for curing the disease of the plant.

An object of the invention is a method for the diagnosis of the vine disease Flavescence dorée comprising the step of
a) identifying one or both of nucleotides in position 108 and 171 of SEQ ID NO 5, and/or of one or both their complementary nucleotides in the sequence complementary to SEQ ID NO 5, in a vine plant sample comprising phloem wherein the presence of a C residue in position 108 of SEQ ID NO 5 and/or of a G residue in its complementary nucleotide in the sequence complementary to SEQ ID NO 5 and/or of a G residue in position 171 of SEQ ID NO 5 and/ or of a C residue in its complementary nucleotide in the sequence complementary to SEQ ID NO 5, indicates the presence of at least one of the phytoplasma strains associated with the vine disease Flavescence dorée in the sample.

According to the present description, the phrase "..its complementary nucleotide in the sequence complementary to SEQ ID NO.." can be also defined as "its complement" meaning the complementary position with respect to a nucleotide position in the sequence of reference, in the sequence complementary to the sequence of reference, hence, where the phrase "...its complementary nucleotide in the sequence complementary to SEQ ID NO..." or "nucleotide N in the corresponding position in the sequence complementary to SEQ ID NO.." where N can be any nucleotide or a similar phrase meaning the same with a slightly different wording can be substituted by the terms "its or the complement".

The sample according to the present description can be any sample obtainable from a vine plant, provided that the sample comprises phloem of the plant due to the presence of the phytoplasmas being limited to this plant fluid.

The skilled person would know how to obtain such sample; when the sample is taken from a plant it is possible to obtain the fluid directly from the leaf veins. Phloem samples are also available from vine shoots or from the fruits or from the seeds or from the rootstock. Protocols for the collection of phloem-comprising samples are available to the skilled person without need of further details in the present description (Pasquini, et. al, 2001).

In the method of the invention the phloem obtainable by standard methods from the sample can be used as such, or an extraction of the total nucleic acids (TNA) can be carried out with standard methods (by way of example the method described in Angelini, et al. 2001 Flavescence dorée in France and Italy: occurrence of closely related phytoplasma isolates and their near relationships to Palatinate grapevine yellows and an alder yellows phytoplasma. Vitis 40:79-86. (Page 80 column 2), or by 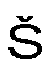 eruga Musi 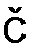 et al. 2008, page 121.

The identification of one or both of the nucleotides in position 108 and 171 of SEQ ID NO 5 (and/or of one or more of their complementary nucleotides in the sequence complementary to SEQ ID NO 5) can be carried out with any known technique suitable for the detection of a known SNP, the presence of nucleotide C in position 108 of SEQ ID NO 5 and/or of the nucleotide G in the corresponding position in the sequence complementary to SEQ ID NO 5 and /or of nucleotide G in position 171 of SEQ ID NO 5 and/or of the nucleotide C in the corresponding position in the sequence complementary to SEQ ID NO 5, indicates the presence in the sample of the phytoplasma associated with the FD vine disease.

By way of example, the identification of step a) may comprise a first step of PCR amplification of one or more fragment comprising one or both of the SNPs of interest and a subsequent analysis of the amplified fragment(s) either by assessing the mass of the amplified fragment(s) and hence defining the base pair present in the position of interest or by merely sequencing the fragment(s) or by using the amplified fragment(s) for other detection techniques as some of the techniques exemplified below.

If both SNPs are amplified each in a different fragment, a different amplicon size can be selected for the two fragments in order to easy the detection.

In an embodiment of the invention, the identification step a) may comprise a first step wherein a real-time PCR (also RT-PCR), Ligation, Allele Specific Hybridization, primer extension, invasive cleavage or sequencing reaction is carried out, and a second step wherein the product obtained in the first step is detected by monitoring the light emitted by said product, by measuring the mass of said products, by monitoring the radioactivity emitted by said product, and/or by sequencing said product.

It will be readily understood by the skilled person that any other suitable protocol and technique known for the identification of a SNP in a nucleotide sequence can be applied to the method herein described.

Several techniques for SNPs identification known in the art and suitable for carrying out the diagnostic method herein described are summarised in Kwok 2001. Numerous techniques are known in the art for the identification of a given SNP in a nucleic acid, several of them are applicable to a solid support technology such as microtiters or microarrays or chips and are thus suitable for the screening of a high number of samples. When a screening in a vineyard or in a vine nursery has to be carried out, methods for the detection of the SNP of interest that are easily applicable to a large amount of samples are convenient.

When RT PCR is used, it is possible to apply a classic RT PCR protocol available in RT PCR manuals and in laboratory protocols. The methods used to verify the identity of the amplicon(s) produced in real-time PCR are sufficiently powerful to detect small variations between sequences. Variations in sequence, including single nucleotide polymorphisms (SNPs) have been successfully identified in RT PCR assays. One common approach to the detection of sequence variation is to compare melting curves. In general, the effect of base substitutions on the melting kinetics of PCR products is too small to be detected reliably; however, heteroduplexes of relatively long amplicons differing by a SNP can be distinguished from the homoduplexes on the basis of their melting curves.

The melting curves of short fluorescent probes can be used to distinguish between amplicons. This method is sensitive to SNPs, which usually cause a shift in the melting peak of several degrees. The design of primers suitable for RT-PCR is easily achievable with suitable programs available to the skilled person (a possible primer pair is depicted in SEQ ID NO 2 and 4) and the disclosure of SEQ ID NO 1 and of SEQ ID No 5 and of the SNP of interest in position 108 of the same, is sufficient for the skilled person to readily design the RT PCR assay without use of inventive skill or cumbersome experimentation. The same applies for the detection of the SNP of interest in position 171 or of both SNPs. The skilled person will easily design primers and oligonucleotides for the amplification and detection of the SNP in position 171 or for the amplification and detection of a fragment comprising both SNPs.

Real-time PCR may involve the use of fluorescently labelled nucleic acid probes or primers, or DNA-binding fluorescent dyes such as SYBR® Green and others mentioned in the present description, to detect and quantify a PCR product at each cycle during the amplification. If desired, different fluorescent dies can be used for the different SNPs.

Hence, according to the present description, the amplified product(s) or the probes can be labelled with a fluorophore following the manufacturer's instructions when melting curve of short fluorescent probes is analysed.

Many RT PCR approaches employ two different fluorescent reporters and rely on the energy transfer from one reporter (the energy donor) to a second reporter (the energy acceptor) when the reporters are in close proximity. The second reporter can be a quencher or a fluor. A quencher will absorb the energy from the first reporter and emit it as heat rather than light, leading to a decrease in the fluorescent signal. A fluor will absorb the energy and emit it at another wavelength through fluorescence resonance energy transfer (FRET), resulting in decreased fluorescence of the energy donor and increased fluorescence of the energy acceptor. The change in fluorescence is proportional to the accumulation of PCR product.

A common alternative to the melting curve approach is to use hydrolysis (such as TaqMan) probes.

Hydrolysis probes are labelled with a fluorescent dye at the 5'-end and a quencher at the 3'-end, and because the two reporters are in close proximity, the fluorescent signal is quenched. During the annealing step, the probe hybridizes to PCR product synthesized in previous amplification cycles. The resulting probe: target hybrid is a substrate for the 5'→3' exonuclease activity of Taq DNA polymerase, which degrades the annealed probe (3) and liberates the fluor. The fluor is freed from the effects of the quencher, and the fluorescence increases. In an embodiment the identification of the SNP of interest can be carried out by RT PCR using suitable primer pairs and a suitable allele specific oligonucleotide probe (TaqMan ® probe), e.g. an MGB (Minor Groove Binding) probe. The allele-specific "TaqMan probe" may be designed based on the SNP information described above. The 5' end of TaqMan probe is labelled with fluorescence reporter dye R (e.g. FAM or VIC), and at the same time, the 3' end thereof is labelled with quencher Q (quenching substance). Thus, under these conditions, fluorescence is not detectable since the quencher absorbs fluorescence energy. Since the 3' end of TaqMan probe is phosphorylated, no extension reaction occurs from TaqMan probe during PCR reaction.

In an embodiment of the invention, the TaqMan® MGB probes can be labelled with 6-carboxyfluorescein (FAM) at the 5'□ end and with a non-fluorescent quencher (NFQ) with minor groove binder (MGB) at the 3' end.

MGB probes in the present invention disclosed a higher melting temperature (Tm) and increased specificity and were MGB probes were more sequence specific than standard DNA probes, especially for single base mismatches at elevated hybridization temperatures.

All RT PCR reactions may be carried out on an ABI PRISM® 7300 Sequence Detection System (Applied Biosystems) in optical 96-well plates with optical adhesive covers (both Applied Biosystems) using the following cycling conditions: 10 min at 95°C, followed by 40 cycles of 15 s at 95°C and 1 min at 61,5°C, which allowed running of all reactions on the same plate. RT PCR can be performed in a final reaction volume of 25 µL containing 5 µL of sample DNA, 300 nm primers, 250 nm probe and 1 × TaqMan® Universal PCR Master Mix (Applied Biosystems), which includes ROX as a passive reference dye.

Suitable primers and probes can be designed with various algorithms and programs available also on the web or the design thereof can be commissioned via commercially available services. In a non limitative way suitable probes could be a forward primer of 26 nucleotides in length starting at position 72 from 5' to 3' of SEQ ID NO 5, provided herein as SEQ ID NO 2, a TaqMan ® probe of 17 nucleotides in length starting at position 102 from 5' to 3' of SEQ ID NO 1, provided herein as SEQ ID NO 3 labelled e.g. with 6-FAM at the 5' end, and a reverse primer of 25 nucleotides in length starting at position 147 from 5' to 3' of SEQ ID NO 1, provided herein as SEQ ID NO 4. When a TaqMan ® probe is used, the SNP (C/T) of interest is located centrally in said probe.

When SEQ ID NOs 2-4 are used in this assay, the suitable annealing temperature is of about 61- 61,5 °C. Assays using TaqMan ® are well known in the art and full protocols are available to the skilled person, in principle. The 5'-nuclease allelic discrimination assay, or TaqMan assay, is a PCR-based assay for genotyping SNPs. The region flanking the SNP is amplified in the presence of two allele-specific fluorescent probes. The probes do not fluoresce in solution because of a quencher at the 3' end. The presence of two probes allows the detection of both alleles in a single tube. Moreover, because probes are included in the PCR, genotypes are determined without any post-PCR processing.

Techniques suitable for carrying out the method herein disclosed comprise also Allele Specific Hybridization also known as ASO (allele specific oligonucleotide hybridization). This protocol relies on distinguishing between two DNA molecules differing by one base (i. e. the SNP of interest) by hybridization. The TNA obtained by the phloem sample is amplified with suitable PCR primers designed in order to amplify a region of SEQ ID NO 5 comprising one or both of nucleotides 108 and 171, in a convenient embodiment, the PCR amplicons are fluorescence labelled. The fragments obtained by PCR are than applied to immobilized oligonucleotide fragments of SEQ ID No 1 comprising the SNP or the SNPs of interest. After suitable hybridization and washing conditions, fluorescence intensity is measured for each SNP oligonucleotide. Due to the fact that a SNP is analysed, stringent hybridization and washing conditions will have to be used, where by stringent the skilled person will understand conditions that will allow only 100% matched hybridised sequences to remain in the double strand form. Typical means for regulating stringency of a hybridisation protocol are salt concentration (the highest the concentration the lowest the stringency) and/or formamide concentration (the highest the %, the highest the stringency) and/or temperature (the highest the temperature, the highest the stringency). Stringency conditions suitable for identification of SNPs by RT-PCR are known in the art and published on standard protocols for this reaction.

This kind of identification can be carried out also in a solid phase, i.e. on a solid support (defined herein after) such as microtiter, microarray chip and the like, allowing the screening of a large number of samples.

In a particular embodiment, microarrays will be used. The microarray slide is a very powerful diagnostic tool capable to identify, in a single experiment, the presence/absence of a high number of targets. The chips take advantage of an important DNA property, which is the complementary bases match (the T matches with the A and the G with the C) in its structure. The diagnostic technique consists of a luminous signal (emitted by a fluorophore at different wave lengths) in correspondence to the hybridization between the target fragment labelled with the fluorophore and the corresponding probe bound to the microarray slide. This binding, with subsequent light emission, shows that in the group of analysed probes a DNA fragment complementary to the probe that is "lighted" is present and, consequently, allows to know the identity of the target fragment. There are several well known techniques for labelling the nucleic acid analysed (direct, indirect) through the creation of link with fluorophores emitting emit light at different wave lengths (usually in the red and in the green). The probe on the microarray slide is not bound to the fluorophore, therefore the microarray slide that is not hybridised, when observed under a confocal scanner does not show any luminous signal.

Prior to the hybridisation on the microarray a PCR amplification of the target DNA, is carried out in order to both increase the target DNA amount which will easy the detection of the signal, and in order to label the target DNA for the detection on the microarray. The labelling can be carried out with any labelling molecule commonly used for microarrays detection.

On the array, although one copy only could be spotted on the chip, preferably several copies of the probe comprising a fragment of SEQ ID NO 5 wherein a C is present in the position corresponding to position 108 of SEQ ID NO 1 (and/or a G is present in the position corresponding to position 108 SEQ ID NO 5 in the sequence complementary to SEQ ID NO 5) and/or wherein a G is present in the position corresponding to position 171 of SEQ ID NO 5 (and/or a C is present in the position corresponding to position 171 SEQ ID NO 5 in the sequence complementary to SEQ ID NO 5) (i.e. the corresponding fragments of SEQ ID NO 1), can be placed per spot in order to increase the signal on the microarray when the phytoplasma associated to FD is present in the sample analysed and amplified.

The PCR amplification reaction usually requires the use of specific primer pairs for the amplification of different regions of the sequence of interest.

It is evident that the region amplified will have to comprise the nucleotide corresponding to one or both of the nucleotides 108 and 171 of SEQ ID NO 5 (or one or both of the nucleotides complementary to nucleotide 108 and 171 of SEQ ID NO 5 in the sequence complementary to SEQ ID NO 5) i.e. the positions of the discriminating SNPs, in the amplicon(s).

In the present description, fragments comprising the nucleotide corresponding to nucleotide 107 of SEQ ID NO 1 (or the nucleotide complementary to nucleotide 107 of SEQ ID NO 1 in the sequence complementary to SEQ ID NO 1) are fragments wherein the nucleotides upstream and downstream the said nucleotide, perfectly match with the nucleotides upstream and downstream the said nucleotide in SEQ ID NO 1 or in its complementary sequence when the nucleotide complementary to nucleotide 107 is considered.

By perfectly match it is intended that a 100% alignment between SEQ ID NO 5 or SEQ ID NO 1 (or their complementary sequence) and its fragment according to the present description is present.

The design of the primers for the amplification can be carried out with any suitable program available to the skilled person (non limiting examples: Primer Premier, that can be used to design primers for single templates, alignments, degenerate primer design, restriction enzyme analysis, contig analysis and design of sequencing primers; AlleleID and Beacon Designer can design primers and oligonucleotide probes for complex detection assays such as multiplex assays, cross species primer design, species specific primer design and primer design to reduce the cost of experimentation; PrimerPlex is a software that can design ASPE (Allele specific Primer Extension) primers and capture probes for multiplex SNP genotyping using suspension array systems such as Luminex xMAP® and BioRad Bioplex, Primer Express) or by commercially available services for oligo design.

Starting from SEQ ID NO 1 or 5 herein disclosed and knowing that the fragment to be hybridised on the microarray will need to comprise one or both of nucleotides 108 and 171 of SEQ ID NO 1 or 5, the skilled person will easily know how to provide suitable primers for the amplification. A nucleotide strand complementary to the amplified sequence will be spotted on the microarray for the subsequent hybridisation. On the microarray, control fragments can be spotted in order to verify the efficiency of the method used.

In all the methods for carrying out the identification of the SNPs, when SEQ ID NO 5 is mentioned it is evident that the same methods apply automatically for those embodiments to SEQ ID NO 1 as SEQ ID NO 1 is comprised in SEQ ID NO 5. The skilled person can hence read SEQ ID NO 5 instead of SEQ ID NO 1 for all the embodiments of this specification relating to the identification of FD associated phytoplasma.

Suitable primers for the amplification of the fragment comprising the SNP in position 108 to be hybridised on the array are also the primers having SEQ ID NO 2 and SEQ ID NO 4 herein provided as exemplifying primers.

The design of primers for the amplification of a fragment comprising the SNP in position 171 is easily achievable for the skilled person starting from SEQ ID NO 5 or 1 as well as the design of primers for the amplification of a fragment comprising both the SNPs of interest.

The hybridisation conditions for the allele specific hybridisation technique are available in the art; an example of suitable conditions can be 16 hours of hybridisation at 48°C (25% of formamide).

By way of example, the microarray hybridisation could be carried out following a standard protocol as follows, wherein some step may be omitted, modified or added by the skilled person without the use of inventive skill:

### Microarray hybridization and wash conditions:

### Prehybridisation:

1. Prehybridisation buffer: 5x SSC, 0.1% SDS and 1% BSA. Heat to about 50°C while stirring; 2. Slides to be analyzed are placed in a staining jar; prehybridisation buffer is added, and incubation is carried out at about 48°C for 45-60 min while stirring; 3. The slides are washed by dipping up and down approximately 10 times in two different staining jars of deionised water. Excess water is removed by shaking the slide rack up and down two times; 4. The slides are then dipped in an up and down motion approximately 10 times at room-temperature in isopropanol and spun dried. The slides are used immediately after prehybridisation (less than 1 hr) as hybridization efficiency decreases rapidly if the slides are allowed to dry for more than that time.

### Hybridization:

5. 2X hybridization buffer: 50% formamide, 10X SSC and 0.2% SDS. Incubate the solution until it reaches 48°C; 6. The labelled mixtures are resuspended in 9 µl water, and heated to 95°C for 3 min to denature, and are centrifuged at maximum angular velocity for 1 min.; 7. The following is added to each tube in order to block non-specific hybridization. Make a master-mix with the following ingredients for each tube:
- Calf Thymus DNA (1µg/µL) 8µl
- poly(A)-DNA (10mg/mL) 2µl
- yeast tRNA (4mg/mL) 2µl

8. Then, about 21 µl 2X hybridization buffer that has been pre-heated to 48°C is added to the target mixture, mixed well, and centrifuged. The samples are kept at 48°C until placed on the slide; 9. The labelled target is applied to a prehybridized microarray slide and covered with a 22 x 60 mm glass cover slip; 10. The slide is placed in a sealed hybridization chamber (Corning. Acton, MA), and 12 µl water is added to the small reservoirs at each end of the chamber; 11. The sealed chamber is placed in a 48°C water bath and incubated for 40-60hr (2).

### Post-hybridization washes:

12. The array is removed from the hybridization chamber with care taken not to disturb the coverslip; 13. The slide is placed in a rack for a staining dish containing 1X SSC, 0.1% SDS, and 0.1 mM DTT at about 48°C; 14. The coverslip is gently removed while the slide is in solution and agitated for 15 min.; 15. The slides are transferred to a staining dish containing 0.1X SSC, 0.1% SDS, and 0.1 mM DTT at about 48°C and agitated for 5 min.; 16. Repeat step 15 two more times; 17. The slides are transferred to a staining dish containing 0.1X SSC and 0.1 mM DTT at room-temperature and agitated for 5 min.;18. Repeat step 17 an additional time; 19. Slides are spun dried.

The exemplified conditions above are a standard protocol based on which the skilled person will be capable to apply all suitable modifications for carrying out the method of the invention without use of inventive skill.

Suitable labelling for the examples above, and for all the following examples where fluorescence is used for the detection, can be carried out, e.g. with Cy2, Cy3, Cy3.5, Cy5, Cy5.5, Cy7, Fluorescein, 6-Fam, Hex, Tet, Tamra, Joe, Rox, IRDyeTM700, IRDyeTM800, Dyomics Dyes, Atto Dyes or any other suitable commercial dye following the manufacturer's instructions.

Another possible technique for carrying out the method of the invention is by the single base extension (SBE) approach.

According to this technique the target region is amplified by PCR followed by a single base sequencing reaction using a primer that anneals one base inside of the polymorphic region. Several detection methods have been described. One can label the primer and apply the extension products to gel electrophoresis. Or the single base extension product can be broken down into smaller pieces and measured by Mass Spectrometry. The most popular detection method involves fluorescence labeled, dideoxynucleotide terminators that stop the chain extension.

As known by the skilled person, primer extension is a very robust allelic discrimination mechanism. It is highly flexible and requires the smallest number of primers/probes. Probe design and optimization of the assay are usually very straightforward. As for the method described above, the skilled person, starting from the sequence and the SNPs herein disclosed can easily and readily design suitable probes merely using commonly available software for probe design. Given the shortness of SEQ ID NO 5 and 1 and the one or maximum two SNPs to be identified, the design of the suitable probes will not be particularly difficult or toilsome and will not require the use of inventive skill, being the mere application of standard procedures or even commercially available services for probe design sufficient. There are numerous variations in the primer extension approach that are based on the ability of DNA polymerase to incorporate specific deoxyribonucleosides complementary to the sequence of the template DNA. However, they can be grouped into two categories: the first is a sequencing (allele-specific nucleotide incorporation) approach where the identity of the polymorphic base in the target DNA is determined; the second is an allele-specific PCR approach where the DNA polymerase is used to amplify the target DNA only if the PCR primers are perfectly complementary to the target DNA sequence.

In the sequencing approach, one can either determine the sequence of amplified target DNA directly by mass spectrometry or perform primer extension reactions with amplified target DNA as a template and analyze the products to determine the identity of the base(s) incorporated at the polymorphic site (allele specific nucleotide incorporation). Various ways have been devised in the art for primer extension product analysis in homogeneous assays.

In the allele-specific PCR approach, one relies on the DNA polymerase to extend a primer only when its 3' end is perfectly complementary to the template.

When this condition is met, a PCR product is produced. By determining whether a PCR product is produced or not, one can infer the allele found on the target DNA. Several approaches have been utilized to detect the formation of specific PCR products in homogeneous assays, e.g. based on melting curve analysis, or based on hybridization of target specific probes. A variation of this approach is the allele-specific primer extension. Here, the PCR product containing the polymorphic site serves as template, and the 3' end of the primer extension probe consists of the allelic base. The primer is extended only if the 3' base complements the allele present in the target DNA. Monitoring the primer extension event, therefore, allows one to infer the allele(s) found in the DNA sample.

SBE can also be easily carried out on microarrays using the well known SBE-TAG technique. Protocols suitable for carrying out this embodiment of the invention are described in manuals such as, by way of example, in "DNA microarrays: a molecular cloning manual" by David Bowtell, Joseph Sambrook, Protocol 6, pages 403-420, herein incorporated by reference. In protocol 6 of the above mentioned manual all the teachings that are necessary to the skilled person for the design of this embodiment of the invention, from primer selection and design to buffers, is described in detail. The skilled person can hence easily carry out this SBE-tag embodiment of the invention starting from the teachings of the present description without use on inventive skill and without cumbersome preparations. Another protocol suitable for a SBE identification of the SNP of interest on microarrays is the Affymetrix tag array, the protocol being also available on manuals such as the manual mentioned herein above by Bowtell and Sambrook, described in detail in Protocol 7, pages 421-428 herein incorporated by reference.

A further suitable technique for the identification of the SNP(s) of interest is the ligation technique. In the Allele Specific Oligonucleotide Ligation, by designing oligonucleotides complementary to the target sequence, with the allele-specific base at its 3'-end or 5-'end, one can determine the genotype of the PCR amplified target sequence by determining whether an oligonucleotide complementary to the DNA sequencing adjoining the polymorphic site is ligated to the allele-specific oligonucleotide or not. This assay relies on the fact that DNA ligase is an enzyme that is highly specific in repairing nicks in the DNA molecule. When two adjacent oligonucleotides are annealed to a DNA template, they are ligated together only if the oligonucleotides perfectly match the template at the junction.

Allele-specific oligonucleotides can, therefore, interrogate the nature of the base at the polymorphic site. One can infer the allele(s) present in the target DNA by determining whether ligation has occurred. Ligation has the highest level of specificity and it is the easiest to optimize among all allelic discrimination mechanisms, but it is the slowest reaction and requires the largest number of modified probes. However, ligation as a mechanism has the potential of genotyping without prior target amplification by PCR. This can be accomplished either by the ligation chain reaction (LCR) or by the use of ligation (padlock) probes that are first circularized by DNA ligase followed by rolling circle signal amplification.

Also the ligation technique is applicable on microarrays, in this case, the technique is called more specifically Ligation Detection Reaction - Universal Array (LDR-UA) and allows the detection of Single Nucleotide Polymorphisms (SNPs) on DNA molecules. The LDR-UA technique takes advantage of two different probes, called Common Probe (CP) and Discriminating Probe (DP), which are designed to anneal juxtaposed on target single strand DNA. DP anneals on the DNA at the 5' end of CP. The two probes can be ligated by a thermo-stable ligase such as the Pfu Ligase. If the complementarity between the 3' end of the DP and the target DNA is not perfect, the ligation reaction is compromised. For this reason the last base at the 3' end of the DP is also called discriminating base.

The system requires that the probes carry the following modifications:
- Each DP must be labelled with a distinct fluorophore at its 5' end.
- The CP must be phosphorylated at its 5' end and must be extended at its 3' end with a "cZIP Code" sequence, that is the complementary and inverse of the "Zip Code" sequence spotted on the Universal Array. Every CP corresponds to a different "ZIP Code".

The ligation product is hybridized to the UA. Each UA spot is composed of different "Zip Code" DNA sequences that capture the corresponding "cZIP Code" sequences at the CP 3' end. The CP-DP ligation event positions the fluorophore at the 5' end of the DP on the corresponding UA spot that is visualized by the subsequent scanning of the UA. The signal from a spot therefore indicates the perfect match between the DP and the target DNA.

Again, for the ligation techniques, the design of probes starting from SEQ ID NO 5 or 1 and knowing that the SNPs of interest lie in position 108 and 171 of SEQ ID NO 5 or 1 renders the selection of the primers simple for the skilled person. CP and DP (the term extended also to the ligation technique when carried out not on solid supports) can be ordered to commercially available services specialised for the design and construction of this kind of probes. For the aims of the method herein described, it will not even be necessary to design a DP for each allelic form of the SNPs, as the sole aim is the identification of a C (or G in the case of the complementary strand) in position 108 of SEQ ID NO 5 and/or of a G (or C in the case of the complementary strand) in position 171 of SEQ ID NO 5.

The DP probe(s) specific for the T (A in case of the complementary strand) allele in position 108 of SEQ ID NO 5 and/or for the A (T in case of the complementary strand) allele in position 171 of SEQ ID NO 5 could be provided labelled with a different colour for the sake of completeness of the method.

In a yet further embodiment, the identification of the SNP(s) in position 108 and/or 171 of SEQ ID NO 5 or 1 can be carried out by the invasive cleavage technique as described, e.g. by Wilkins Stevens P. et al 2001.

As above indicated, also this technique is known to the skilled person as a suitable approach for the identification of SNPs.

The invasive cleavage assay is a probe cycling, signal amplification reaction used for detection of single nucleotide polymorphisms (SNPs). The reaction requires two synthetic oligonucleotides, called the 'upstream oligonucleotide' and 'probe', that anneal to the target sequence with an overlap of 1 nt. This creates a bifurcated overlapping complex that resembles a structure generated during strand displacement DNA synthesis. Structure-specific 5'-nucleases, whose primary cell function is believed to be processing of Okazaki fragments, cleave the bifurcated substrate at the site of the overlap, releasing the 5'-arm and one base paired nucleotide of the probe. The cleaved 5'-arm serves as a signal indicating the presence of target in an analyzed sample. By performing the invasive reaction at the probe melting temperature (Tm), multiple cleavage events can be achieved for each target. Typically, an invasive signal amplification reaction generates 30-50 cleaved probes/target/min, resulting in 103-104-fold signal amplification in a 1-3 h reaction. The unique ability of 5'-nucleases to specifically cleave the overlapping substrate can be utilized for detection of single base mutations. To detect a particular nucleotide in the target sequence, the upstream oligonucleotide and probe are designed to create overlap at this nucleotide, ensuring efficient cleavage of the probe. A substitution at this nucleotide position eliminates the overlap and dramatically reduces the cleavage rate, resulting in mutation discrimination of at least 300:1. Such discriminatory power makes the invasive cleavage assay an excellent tool for identification of SNPs.

Signal detection can be carried out by electrophoresis, microplate (microtiter) enzyme-linked immunosorbent assay (ELISA), matrix-assisted laser desorption/ionization time-of-flight (MALDI-TOF) mass spectrometry methods, and fluorescence resonance energy transfer (FRET) methodology.

The invasive cleavage assay, as the other techniques mentioned above, is adaptable to a solid phase format presenting the possibility of analyzing multiple SNPs or multiple samples for a single SNP in parallel. SNP detection using the invasive cleavage reaction can be performed in 96-well microplates with nanogram amounts of DNA per SNP.

Furthermore, the SNP can be identified by the SniPer method that allows discriminating alleles by examining the presence or absence of amplification by RCA (rolling circle amplification). Briefly, the DNA to be used as a template is linearized. Then, a probe is hybridized to this linearized DNA. When the probe sequence and the sequence of the linearized DNA as a template are complementary to each other and form a complementary strand, the genomic DNA can be converted into a circular DNA through ligation reaction. As a result, RCA of the circular DNA proceeds. On the other hand, when the ends of the probe do not match with the genomic DNA, the DNA is not ligated to become a circular DNA. Thus, RCA reaction does not proceed. Therefore, in Sniper method, a single-stranded probe which anneals with the genomic DNA and is circularizable is designed. This single-stranded probe is called a padlock probe. The sequences of the two ends of this padlock probe are designed so that they correspond to the SNP to be detected. Then, this padlock probe and the genomic DNA are mixed for ligation. If the two ends of the padlock probe and the SNP site of the genomic DNA are complementary to each other, the two ends of the padlock probe are joined by ligation, yielding a circular probe. If the two ends of the padlock probe and the SNP site of the genomic DNA are not complementary to each other, the probe does not become circular. Therefore, only those padlock probes which are complementary to the SNP to be detected become circular and are amplified by DNA polymerase. By detecting the presence or absence of this amplification, SNP may be detected. For the detection, synthetic oligonucleotides which have a fluorescent dye and a quencher at their respective ends and also have a hairpin structure are used.

Finally, a further technique applicable to the diagnostic method herein disclosed, is the amplification of a fragment comprising one or both of the nucleotides 108 and 171 of SEQ ID NO 5 or 1 and sequencing of the same with direct identification of one or both of nucleotides 108 and 171 (and/or of their complementary nucleotides). The sequencing can be readily and easily performed with automated sequencers. When a fragment sequence is carried out, the amplification primers of SEQ ID NO 2 and 4 are suitable also for this technique. It is evident that any primer pair suitable for the specific amplification of all or part of SEQ ID NO 5 or 1 provided that one or both of nucleotides 108 and 171 are comprised in the amplicon obtained (and/or one or both of the corresponding nucleotides in the sequence complementary to SEQ ID NO 5 or 1), or any probe overlapping with one or both of nucleotides 108 and 171 of SEQ ID NO 5 or 1 (and/or one or both of the corresponding nucleotides in the sequence complementary to SEQ ID NO 5 or 1) can be used for carrying out the method of the invention, and that the invention is not limited to primers of SEQ ID NO 2-4.

In all the embodiments described above, where the techniques used allows for the distinction within the two different alleles of the SNP(s) of interest, a control could be carried out by detecting also the allele carrying T (A in case of the complementary strand) in position 108 of SEQ ID NO 5 and/or of an A (T in case of the complementary strand) that are specific for the phytoplasmas of the same taxonomic group and subgroups that are not associated (but confusable in terms of symptoms of the plant to a certain extent) to FD (e.g. phytoplasma associated with EY, ALY and ULW). It is evident that in a kit for carrying out the diagnostic method herein described suitable probes can be provided also for the detection of the allele specific for the EY, ALY and ULW associated phytoplasma.

As stated above, the diagnostic method herein disclosed, can be carried out on solid supports. Suitable solid supports can be a latex bead, a glass slide, a silicon chip, or the walls of a microtiter well. In some cases, marker specific oligonucleotides are placed on the solid support, and the allelic discrimination reaction is done on the support whereas in other cases, generic oligonucleotides are placed on the solid support, and they are used to capture complementary sequence tags conjugated to marker specific probes. When marker specific oligonucleotides are placed on the solid support, the oligonucleotide arrays act as a collection of reactors where the target DNA molecules find their counterparts, and the allelic discrimination step for numerous markers proceeds in parallel. When generic oligonucleotides are placed on the solid support, the arrayed oligonucleotides are used to sort the products of the allelic discrimination reactions (also done in parallel) performed in homogeneous solution. In both cases, the identity of an oligonucleotide on a latex bead or at a particular location on the microarray (on a glass slide or silicon chip) is known, and the genotypes are inferred by determining which immobilized oligonucleotide is associated with a positive signal. The clear advantage of performing genotyping reactions on solid supports is that many markers or, in the present case many samples, can be interrogated at the same time. Besides saving time and reagents, performing numerous reactions in parallel also decreases the probability of sample/result mix-ups.

As previously mentioned, the detection of the product obtained by the first step of the identification step a), e.g. the product obtained by the aforementioned techniques, can be performed by monitoring the light emission, by measuring of the mass, by monitoring of the radioactivity or by sequencing of the said product. According to the present description, said monitoring of the light emission can be carried out by monitoring fluorescence, luminescence, time resolved fluorescence, fluorescence resonance energy transfer, or fluorescence polarisation, said measuring of the mass can be carried out by mass spectrometry and said monitoring of the radioactivity can be carried out with radioactivity sensitive tools. Monitoring light emission is the most widely used detection modality in genotyping, this can be done by measuring or detecting Luminescence, fluorescence, time resolved fluorescence, fluorescence resonance energy transfer (FRET), and fluorescence polarization (FP).

Luminescence is emitted in an ATP-dependent luciferase reaction. When ATP production is coupled with a primer extension reaction, luminescence is observed every time a deoxyribonucleoside is added in the primer extension reaction. Luminescence can be measured with suitable commercial analysers following the manufacturer's instructions (e.g. Applied Biosystems 1700 Chemiluminescent Microarray Analyzer).

Fluorescence can be measured using commercially available fluorescence sensitive imaging devices or measuring devices known in the art and labelled probes or amplified products according to the selected technology for carrying out the method herein described. Suitable fluorophores for labelling the nucleic acids of interest can be selected from the group consisting of Cy2, Cy3, Cy3.5, Cy5, Cy5.5, Cy7, Fluorescein, 6-Fam, Hex, Tet, Tamra, Joe, Rox, IRDyeTM700, IRDyeTM800, Dyomics Dyes, Atto Dyes.

Detection can be carried out following the manufactures instructions. The skilled person is well aware that all techniques indicated above can all be carried out with fluorophores following conventional protocols.

Detection by Time-resolved fluorescence can be made with dyes having a long half life (such as Lanthanides), the fluorescence reading is done sufficiently long after excitation, such that autofluorescence (which has a very short half-life) is not observed. As lanthanides are inorganic compounds that cannot be used to label nucleic acids directly an organic chelator conjugated to the probe must be used to bind the lanthanides in the reaction. Protocols are available for this kind of detection. Also for this kind of detection standard protocols and manufacturers protocols are available to the skilled person.

Fluorescence resonance energy transfer (FRET) occurs when two conditions are met. First, the emission spectrum of the fluorescent donor dye must overlap with the excitation wavelength of the acceptor dye. Second, the two dyes must be in close proximity to each other because energy transfer drops off quickly with distance. The proximity requirement is what makes FRET a good detection method for a number of allelic discrimination mechanisms. Basically, any reaction that brings together or separates two dyes can use FRET as its detection method. FRET detection has, therefore, been used in primer extension and ligation reactions where the two labels are brought into close proximity to each other. It has also been used in the 50 nuclease reaction, the molecular beacon reaction, and the invasive cleavage reactions where the neighbouring donor/acceptor pair is separated by cleavage or disruption of the stem-loop structure that holds them together. Dyes pair suitable for FRET detection are known in the art.

FP can be used in several SNPs detection techniques, commercial systems such as the Perkin Elmer AcycloPrime™-FP SNP Detection System are available to the skilled person.

According to the present description also systems that make no use of fluorescent dyes can be used for the detection of the SNPs, a commonly used system is the mass spectrometry (MS) where the molecular weight of the products formed is measured. In MS detection no labels are needed. High resolution MS can easily distinguish between DNA molecules that differ by only one base in times of milliseconds to analyze each sample. MS can be measured with commercially available mass spectrometers following standard protocols.

A further method requires the use of radiolabels instead of fluorescent labels and in this case radioactivity of the sample is measured in standard ways well known in the art.

Another embodiment of the present invention is a diagnostic kit for the diagnosis of the vine disease Flavescence dorée comprising reagents for the identification of one or both of the nucleotides in position 108 and 171 of SEQ ID NO 5 and/or of one or more of their complementary nucleotides in the sequence complementary to SEQ ID NO 5 in a plant phloem.

The kit of the present invention comprises any or all components enzymes or components necessary (suitable) for an intended assay. Examples of such components include, but are not limited to, labelled and/or non labelled oligonucleotides, polymerases (e.g. Taq polymerase), buffers (e.g. Tris buffer), dNTPs labelled or non labelled, control reagents (e.g. tissue samples, target oligonucleotides for positive and negative controls, etc.), labelling and/or detection reagents (fluorescent dyes such as VIC, FAM), solid supports, manual, illustrative diagrams and/or product information, inhibitors, and packing environment adjusting agents (e.g. ice, desiccating agents). The kit of the present invention may be a partial kit which comprises only a part of the necessary components. In this case, users may provide the remaining components. The kit of the present invention may comprise two or more separate containers, each containing a part of the components to be used. For example, the kit may comprise a first container containing an enzyme and a second container containing an oligonucleotide. Specific examples of the enzyme include also a structure-specific cleaving enzyme, ligases or other enzymes for use in the techniques for identification and detection described above contained in an appropriate storage buffer or a container. Specific examples of the oligonucleotide include nucleotides for the RT-PCR, nucleotides for the extension technique described above, nucleotides the ligation technique described above, oligonucleotides for the LDR-UA technique described above, oligonucleotides for the invader technique described above, probe oligonucleotides for the hybridisation technique described above, target oligonucleotides for use as control, and the like.

The oligonucleotides can be labelled according to the technique selected.

Alternatively, ore or more reaction components may be provided in such a manner that they are pre-divided into portions of a specific amount. Selected reaction components may also be mixed and divided into portions of a specific amount. It is preferred that reaction components should be pre-divided into portions and contained in a reactor. Specific examples of the reactor include, but are not limited to, reaction tubes or wells, or microtiter plates. It is especially preferable that the pre-divided reaction component should be kept dry in a reactor by means of, for example, dehydration or freeze drying.

The kit of the invention may further comprise solid supports wherein oligonucleotides for the identification of one or both of the nucleotides in position 108 and 171 SEQ ID NO 5 and/or of one or both of their complementary nucleotides in the sequence complementary to SEQ ID NO 5 are anchored in known positions on said support in one or more copy per position.

The anchorage of the oligonucleotides/probes as described above in the section related to the techniques suitable for carrying out the method will be anchored to the solid support by standard techniques selected depending on the support chosen. The kit of the invention may comprise labelled probes corresponding and/or complementary to a region of SEQ ID NO 5 upstream and/or downstream of one or both of the nucleotides in position 108 and 171 of SEQ ID NO 5 and/or overlapping a region comprising one or both of the nucleotides in position 108 and 171 of SEQ ID NO 1.

Upstream and downstream oligonucleotides may be directly adjacent to one of the nucleotides 108 or 171 of SEQ ID NO 1 or to its complementary nucleotide in the sequence complementary to SEQ ID NO 1 e.g. in case of kits for identification by ligation or by other techniques as exemplified above requiring probes directly adjacent to the SNP; or may be non directly adjacent to the SNP nucleotide when the kits are for identification with techniques as exemplified above that to not require probes directly adjacent to the SNP.

All the probes and oligonucleotides of the invention (e.g. probes described for the method and for the kit) fall within the definition of fragments of SEQ ID NO 1 or of its complementary sequence and will be of a dimension comprised between about 8 to about 100 nucleotides, normally of a dimension comprised between about 15 to about 50 nucleotides. When the fragments and probes do not comprise one or both the SNPs of interest they also fall within the definition of fragments of SEQ ID NO 5 or of its complementary sequence and will be of a dimension comprised between about 8 to about 100 nucleotides, normally of a dimension comprised between about 15 to about 50 nucleotides. The fragments, on the other hand, include also fragments that are larger than the oligonucleotides and probes, could be represented e.g. by amplicons and can span from the dimensions of the oligonucleotides and probes indicated above, to a length equal to the length of SEQ ID NO 1 minus 1 nucleotide.

According to a previously exemplified embodiment of the invention, the probes and oligonucleotides of the kit may be labelled with a fluorophore, e.g. with a fluorophore selected from the group consisting of Cy2, Cy3, Cy3.5, Cy5, Cy5.5, Cy7, Fluorescein, 6-Fam, Hex, Tet, Tamra, Joe, Rox, IRDyeTM700, IRDyeTM800, Dyomics Dyes, Atto Dyes.

The skilled person will understand that selection of further dies for labelling the probes and oligonucleotides falls within the normal skills of a technician skilled in molecular genetics.

The kit may also comprise the vector described either in the positive or negative control embodiment as described above, or may comprise both a positive and a negative control i.e. the positive control will comprise FD associated phytoplasma's specific SNP and/or SNPs disclosed in the present description (i.e. from SEQ ID NO 1) and the negative control will comprise at the disclosed SNP or SNPs site/s a sequence from SEQ ID NO 5 when SEQ ID NO 5 is not SEQ ID NO 1. These controls will allow verifying the correct functioning of the kit or of the method herein described without need of phytoplasma nucleic acids as controls.

By way of example, monoclonal or polyclonal antibodies specifically recognising SEQ ID NO 7 can be prepared according to standard techniques and even purchased by companies specialised in antibody preparation starting from SEQ ID NO 7. The antibody can be produced according to any known standard technique such as described also in the monoclonal and polyclonal production related chapters in the manual "Basic Methods in Antibody Production and Characterization" edited by G.C. Howard and D.R. Bethell, ed., CRC Press, 2001 or in other commonly used laboratory manual and the identification of FD associated phytoplasma can be carried out by immunological assays on vine phloem samples where cell lysis has been carried out to expose the L14 ribosomal protein of SEQ ID NO 7 expressed inside the infected cell.

Therefore, by way of example, the method for the detection of FD associated phytoplasma in a vine sample could be carried out, wherein a primary antibody specifically recognising SEQ ID NO 7 and a labelled secondary antibody specifically recognising the primary antibody are used in an immunodetection assay on a vine phloem sample wherein cell lysis has been carried out.

### EXAMPLES

### rpl14 isolation

5 phytoplasma strains (EY1, ULW, ALY, FD92 and FD-C) belonging to group 16SrV were used in this work.

FD92 and FD-C represented two strains of Flavescence dorée, whereas EY1 and ALY are two strains not associated with FD, but sporadically present in grapevine.

DNA extraction: Total nucleic acids (TNA) were extracted as previously described (Angelini et al., 2001). 1g of fresh leaf of periwinkle and grapevines veins was homogenized in CTAB buffer 3% (3% CTAB, 100mM Tris-HCl pH8, 10mM EDTA, 1.4 M NaCl, 0,1% 2-mercaptoethanol). 1ml of solution was transferred to an 1,5ml tube and incubated for 20 min at 65 °C. Then, an equal volume of chloroform was added and, after centrifugation, the upper phase containing DNA was precipitated with an equal volume of isopropanol and collected by centrifugation. The DNA pellet was washed with 70% ethanol, dried and dissolved in 100ul of TE (10mM Tris, 1mM EDTA, pH 7.6).

In order to obtain the partial sequence of S10-spc operon, undiluted extracted DNA was amplified by using the forward primer rpF1(V), positioned in the 3' end of rps19 gene (Lee et al., 1998) and the reverse primer FD9r, positioned in the 3' end of secY gene (Martini et al., 2002). The rpF1(V)/FD9r amplicons had an expected size of 9Kbp, approximately. Direct PCR products were diluted 1:50 in sterile water and used in nested PCR assays performed by means of the forward primer rp(V)F1A (Lee et al., 2004) and the reverse primer FD9r2 (Martini et al., 2002), that amplified a product of an expected size of 8Kbp, approximately, comprised between rpl22 and secY gene on S10-spc operon.

PCRs were performed employing the Platinum® Taq DNA Polymerase High Fidelity (Invitrogen, Germany). PCR mixture (total volume 25µl) contained DNA template (1µl of undiluted extracted DNA for dPCR, and 2µl of 1:50 diluted dPCR products for nPCR), 0.25mM each dNTP, 0.4µM each primer, 1.5U of Platinum® High Fidelity. For PCR amplification, 36 cycles were conducted in an automated T3000 Thermocycler (Biometra GmbH, Goettingen, Germany), at the following conditions: 45sec at 94°C (denaturation), 30sec at 56°C (annealing), 9min (dPCR) or 8min (nPCR) at 68°C (extension), and 10min at 72°C (extension of final cycle). DNA extracted from healthy periwinkle plants and PCR reaction mixture devoid of DNA was used as negative controls. PCR products (5µl) were analyzed by electrophoresis in 1% agarose gel, stained with ethidium bromide, and visualized with a UV transilluminator.

The PCR product rpF1A/FD9r2 covering the S10-spc operon region between the rpl22 and secY genes, was sequenced. A sequence-walking strategy was performed by means of 30 intermediate primers designed on the in progress sequence. Reconstruction of the 6,3Kb amplicon sequence was obtained by using the Contig Assembly Program (CAP) of the software Bioedit version 7.0.0 (http://www.mbio.ncsu.edu/BioEdit/bioedit.html).

In order to search the Open Reading Frames (ORFs) and to deduce the sequences of the codified proteins, our entire sequence was analyzed by using the ExPasy Proteomics Server (http://us.expasy.org/tools/dna.html) (Gasteiger et al., 2003).

### Diagnostic assay design

Sequenced amplicons were then compared with each other in order to identify sequence variation that would allow the identification of regions suitable for design of diagnostic assays.

The sequence alignment was performed with software ClustalW, high sequence homology between different isolates examined was observed. In particular, we have identified a nucleotides common to both FD (FD92 and FD-C) that differentiate them from other isolates belonging to the group 16SrV (Fig1). This nucleotide was located in rpl14gene (381 bp).

One portion of this gene was submitted in Primer Express software, to design an assay specific for FD on base of this SNP identified.

We have identified one nucleotide fragment of 100bp which includes two primers and one probe suitable to detect FD phytoplasma.

### Assay optimization Specificity and Sensitivity

To obtain the specificity to FD with the primers of SEQ ID 2-4 we have modified the conventional thermal profile of PCR. Usually, annealing and extension are performed to 60 °C, however if the reaction was conducted at this T° no specificity to FD was observed. Therefore, we raised the T° to 61,5 °C to achieve more specificity. In fact, when we worked in this condition of PCR reaction none amplification in negative control (EY1 - ALY) was observed. (Tab. 1). In Tab 1 the differences between the same assay conducted to 61,5 °C (A) and 60 °C (B) are shown.

It is important to underscore two main differences: i) at 61,5 °C no negative control showed fluorescent signal, this confirmed the specificity of the assay to FD ii) however, at 60 °C we have observed an increased sensitivity of the assay for the grapevine sample infected by FD, as shown in the column of ΔCt value (difference between assay A and B). In 7 samples, the ΔCt value was 5-fold higher in A than B, however the negative control EY and ALY showed amplification signal. In fig 2 an amplification plot example is described. The results are depicted in Table 1 below.

**Table 1. Comparison between FD66 probe assay - conducted to 61,5 °C (A) and 60 °C (B) - , PCR/RFLP analyses and serological (E.L.I.S.A.) tests on leaf samples collected from diseased and healthy vines.**

| Ct-average | | | | | | | |
|---|---|---|---|---|---|---|---|
| **Type of sample** | **n°** | **Sample code** | **A** | **B** | **Δ Ct** | **PCR/RFLP** | **ELISA** |
| | 1 | 136 | 31,17 | 30,07 | 1 | + | - |
| | 2 | 137 | 34,46 | 34,41 | 0 | + | - |
| | 3 | 143 | 32,36 | 27,25 | 5 | + | - |
| | 4 | 144 | 32,49 | 26,43 | 6 | + | - |
| | 5 | 145 | 32,20 | 29,62 | 3 | + | - |
| | 6 | 146 | 34,56 | 28,09 | 6 | + | - |
| Grapevine leafs | 7 | 147 | 32,20 | 28,36 | 4 | + | - |
| | 8 | 148 | 33,03 | 25,96 | 7 | + | - |
| | 9 | 150 | 34,17 | 29,84 | 4 | + | - |
| | 10 | 151 | 35,03 | 33,96 | 1 | + | - |
| | 11 | 152 | 32,96 | 26,88 | 6 | + | - |
| | 12 | 153 | 31,23 | 27,97 | 3 | + | - |
| | 13 | 154 | 32,04 | 29,62 | 2 | + | - |
| | 14 | 155 | 34,31 | 28,69 | 6 | + | - |
| | 15 | 257 | 32,33 | 24 | 8 | + | - |
| Positive control | FD | | 18,00 | 16 | 2 | + | +(very faint) |
| Negative control | EY | | negative | 33 | - | + | - |
| | ALY | | negative | 33 | - | + | - |
| | **AY** | | negative | negative | | negative | nt |
| | **STOL** | | negative | negative | | | negative |

legenda:
nt - not tested

Table 1 shows the differences between the same assay conducted at 61,5 °C (A) and 60 °C (B) and with the commercial ELISA kit (SEDIAG^{®}, France). 15 samples were tested to evaluate the sensitivity and specificity of the FD66 RTPCR assay. In condition A, the assay was conducted at 61,5 °C and we can observe high specificity for FD phytoplasma but less sensitivity. In condition B, the assay was conducted at 60 °C, and we have observed an increased sensitivity of the assay for the grapevine sample infected to FD, but a poor specificity for FD phytoplasma. The difference in the sensitivity of the two conditions was calculated using the ΔCt value. No phytoplasma was detected using ELISA test.

### Sample preparation for E.L.I.S.A. test.

Samples were prepared following the instructions of the manufacturer, and the ELISA test was carried out following the same. Due to the negative results obtained, and to the very weak signal obtained only for the control provided in the kit, different batches of the SEDIAG^{®} Kit 480 were tested with the same results reported in Table 1.

The table shows a comparison between the conventional method currently used (PCR-endpoint) and the diagnostic test here proposed (based on real-time-PCR). We didn't compare the proposed method with the pre-existing (Bianco et al., 2004) because: i) in this paper the target was a PCR product while in the present assay we used TNA and ii) the sequence target in the above mentioned paper was 16Sr DNA gene while the proposed, present, method targets a ribosomal protein gene. The protocol reported in the literature is always based on the presence of a SNP. It can discriminate FD from EY, but, as previously described, two amplification reactions are needed in order to specifically detect the phytoplasma associated with FD. Therefore, the method proposed in the present invention represents a new technology compared to the previous techniques.

### Hybridisation on microarray

The amplified genomic DNA is labelled with the "BioPrime® Total Genomic Labeling System" kit (INVITROGEN - 18097-012) following the manufacturer's instructions. The labelled sample is precipitated by Spin - Vacuum Savant and is subsequently re suspended in the hybridisation solution.

### Microarray hybridisation:

Probes comprising one or both the SNPs region of interest have been blocked on the slide for the detection of the phytoplasma SNP(s) associated with FD disclosed in the present description, in 16 copies per probe. Before the hybridisation reaction, the activation of the slide shall be carried out (glass surface chemistry: EPOXY Surface Coating Slides; spotting buffer: Scott-Nexterion spotting buffer; probe's concentration: 30µM), by the use of a blocking solution (10x Sodium Saline Citrate (SSC), 0.1% Sodium Dodecyl Sulfate (SDS), 0.066 Sodium Tetrahydridoborate (NaBH₄), H₂O up to 50ml). The slide is treated with the blocking solution for 20 minutes to 42°C. Washings are carried out: twice (Sodium Saline Citrate 1x for 5 minutes at room temperature), twice (Sodium Saline Citrate 0.1x for 5 minutes at room temperature).

### Prehybridisation:

A solution consisting of: 5x Sodium Saline Citrate (SSC), 0.1% Sodium Dodecyl Sulfate, 200µg Salmon Sperm DNA, 5x Denhardt's Solution (5g Ficoll, 5g Polyvinyl pyrrolidone, 5g albumin of bovine serum, fraction V, H₂O up to 500ml), H₂O up to 2ml is prepared. The solution must be filtered by 0.2µm filters. The slide area comprising the probes is delimited with a "frame" (Gene Frame 21x22mm and cover slips - AB1043 CELBIO). 11µl of prehybridisation solution are placed within this area and the slide is covered with the cover slip. The slide with the prehybridisation solution is incubated at 42°C for 2 hours.

### Hybridisation:

A solution consisting of: 5X Sodium Saline Citrate (SSC), 0.1% Sodium Dodecyl Sulfate, 25% Formamide, 200µg Salmon Sperm DNA, H₂O up to 2ml is prepared. The solution must be filtered by 0.2µm filters and preheated at 42°C. The sample of the amplified and labelled DNA is re suspended in about 110µl of hybridisation solution. The DNA sample, resuspended in hybridisation solution, is denatured at 95°C. The hybridisation solution is placed in the centre of the "frame" that defines the area comprising the target probes, and the cover slip is placed on this area. The slide is hence incubated to 42°C for 16 hours.

### Post-hybridisation washing:

1^{st} WASHING - in a volume of 50ml solution: 1x Sodium Saline Citrate (SSC), 0.1% Sodium Dodecyl Sulphate (SDS) for 5 minutes to 42°C;
2^{nd} WASHING - in a volume of 50 ml solution: 0.2x Sodium Saline Citrate (SSC), 0.1% Sodium Dodecyl Sulphate (SDS) for 5 minutes to 42°C;
3^{rd} WASHING - in a volume of 50ml solution: 0.2x Sodium Saline Citrate (SSC) for 5 minutes to 42°C;
4^{th} WASHING - in a volume of 50ml solution: 0.2x Sodium Saline Citrate (SSC) for 5 minutes to 42°C.

The slide has been dried by centrifugation to 800 rpm for 5 minutes.

### BIBLIOGRAPHY

- ANGELINI, E. et al 2001 "Flavescence dorée in France and Italy: occurrence of closely related phytoplasma isolates and their near relationships to palatinate grapevine yellows and an alder yellows phytoplasma." Vitis, 40, 79-86.
- ANGELINI, E. et al 2007 "A new Taqman method for the identification of phytoplasmas associated to grapevine yellows by real time PCR assay." Journal of Microbiological Methods. 6 8, 613-622.
- Belli G. et al 1973. "Presenza di una malattia del tipo "Flavescence dorée" in vigneti dell'Oltrepò pavese." Rivista di Patologia Vegetale Ser. IV, 9 (Suppl.): 50-56.
- BIANCO, P.A., et al 2004. "Detection of phytoplasmas associated with grapevine - flavescence dorée disease using real-time PCR". Journal of Plant Pathology, 86(3): 257-261.
- BOTTI S. and BERTACCINI A. 2007 Grapevine yellows in Northern Italy: molecular identification of Flavescence Dorée phytoplasma strains and of Bois Noir phytoplasmas. Journal of applied microbiology. 103: 2325-2330.
- BOUDON-PADIEU, E., 2003. The situation of grapevine yellows and current research directions: Distribution, diversity, vectors, diffusion and control. Extended Abstracts 14th Meeting of the ICVG, September 12-17,2003 Locorotondo (Bari), Italy, 47-53.
- CAUDWELL A.,1957. Deux annèes d'ètudes sur la Flavescence dorèe, nouvelle maladie grave de la vigne. Annales d'Amelioration des Plantes. 12: 359-393.
- CREDI, R., 2005. "A multiple real-time PCR assay for the detection of bois noir and grapevine flavescence dorée phytoplasmas" Atti 3 Incontro Nazionale sulle Malattie da Fitoplasmi, Milano, 2005: 130-131.
- FIRRAO, G. et al 2004. 'Candidatus Phytoplasma', a taxon for the wall-less, non-helical prokaryotes that colonize plant phloem and insects. International Journal of Systematic and Evolutionary Microbiology, 54, 1243-1255.
- DAVIS, R.E., et al 1997. Phytoplasmas associated with grapevine yellows in Israel and Greece belong to the stolbur phytoplasma subgroup, 16SrXII-A. J. Plant Pathol.79:181-187.
- GALETTO, L., et al 2005. "Universal and group-specific real-time PCR diagnosis of Flavescence dorée (16Sr-V), bois noir (16Sr-XII) and apple proliferation (16Sr-X) phytoplasmas from field collected plant hosts and insect vectors". Annals of Applied Biology. 147, 191-201.
- GASTEIGER E, et al ExPASy: The proteomics server for in-depth protein knowledge and analysis. Nucleic Acids Res 2003;31(13):3784-3788.
- HREN, M., et al 2007. Real-time PCR detection systems for Flavescence dorée and Bois noir phytoplasmas in grapevine: comparison with conventional PCR detection and application in diagnostics. Plant Pathology 56, 785-796.
- KWOK 2001, "Methods for genotyping Single Nucleotide Polymorphisms" Annu. Rev. Genomics Hum. Genet. 2001. 2:235-58.
- LEE, I.M., et al, 2000. "Phytoplasma: Phytopathogenic mollicutes" Annu. Rev. Microbiol., 54, 221-255.
- LEE, I.M., et al. 2004. "Classification of phytoplasma strains in the elm yellows group (16SrV) and proposal of 'Candidatus Phytoplasma ulmi' for the phytoplasma associated with elm yellows". International Journal of Systematic and Evolutionary Microbiology, 54(337), 347.
- MAIXNER, M., and W. REINERT. 1999. Oncopsis alni (Schrank) (Auchenorrhyncha:Cicadellidae) as a vector of the alder yellows phytoplasma of Alnus glutinosa (L.) Gaertn. Eur. J. Plant. Pathol. 105:87-94.
- MAIXNER, M., W. REINERT, and H. DARIMONT. 2000. Transmission of grapevine yellows by Oncopsis alni (Schrank) (Auchenorrhyncha: Macropsinae). Vitis. 39:83-84.
- MARTINI M. et al, 2002.- Genetic variability among "flavescence dorée" phytoplasmas from dif- ferent origins in Italy and France.- Molecular and Cellular Probes, 16: 197-203.
- PASQUINI G. et al 2001. Armonizzazione della diagnosi della flavescenza dorata della vite (FD): risultati di una prova comparativa. Atti Progetto POM A32, vol. II, Locorotondo 2001, 921-947.
- POLACEK N AND MANKIN AS, 2005 . The ribosomal peptidyl transferase center: structure, function, evolution, inhibition. Crit Rev Biochem Mol Biol. 2005 Sep-Oct;40(5):285-311.
- 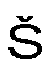 ERUGA MUSIC et al 2008 "Distribution of phytoplasma diseases in the Lombardy poplar tree population of Zagreb urban area". Acta Bot. Croat. 67 (2), 119-130, 2008.
- WILKINS STEVENS P. et al 2001, "Analysis of single nucleotide polymorphisms with solid phase invasive cleavage reactions". Nucleic Acids Research. 2001, Vol. 29, No. 16 e 77.

## Claims

1. A method for the diagnosis of the vine disease Flavescence dorée comprising the step of
a) identifying one or both of the nucleotides in position 108 and 171 of SEQ ID NO 5, and/or their complementary nucleotides in the sequence complementary to SEQ ID NO 5, in a plant sample comprising phloem;
wherein the presence of a C residue in position 108 of SEQ ID NO 5, and/or of a G residue in its complementary nucleotide in the sequence complementary to SEQ ID NO 5 and/or of a G residue in position 171 of SEQ ID NO 5, and/or of a C residue in its complementary nucleotide, indicates the presence of at least one of the phytoplasma strains associated with the vine disease Flavescence dorée in the sample.

2. The method of claim 1 wherein the identification of step a) comprises a first step wherein a PCR, real-time PCR, Ligation, Allele Specific Hybridization, primer extension, invasive cleavage or sequencing reaction is carried out, and a second step wherein the product obtained in the first step is detected by monitoring the light emitted by said product, or measuring the mass of said products, or monitoring the radioactivity emitted by said product, or by sequencing said product.

3. The method of any one of claims 1 or 2 wherein said monitoring of the light emission is carried out by monitoring fluorescence, luminescence, time resolved fluorescence, fluorescence resonance energy transfer, or fluorescence polarisation, said measuring of the mass is carried out by mass spectrometry or heteroduplex analysis, and said monitoring of the radioactivity is carried out with radioactivity sensitive tools.

4. The method of any one of claims 1-3 wherein said method is performed on solid phase format such as microarray or microplate.

5. The method of any one of claims 1-3 wherein said identification is carried out with a RT PCR using a forward primer of SEQ ID NO 2, an allele specific probe of SEQ ID NO 3 and a reverse primer of SEQ ID NO 4.

6. A kit for the diagnosis of the vine disease Flavescence dorée comprising reagents for the identification of the nucleotides in position 108 and 171 of SEQ ID NO 5 or 1 and/or of their complementary nucleotides in the sequence complementary to SEQ ID NO 5 or 1 in a vine plant phloem.

7. The kit of claim 6 further comprising solid supports wherein oligonucleotides for the identification of one or both of the nucleotides in position 108 and 171 of SEQ ID NO 5 or 1 and/or one or both of their of its complementary nucleotide in the sequence complementary to SEQ ID NO 5 or 1 are anchored in known positions on said support in one or more copy per position.

8. The kit of claim 7 further comprising labelled probes and/or oligonucleotides corresponding and/or complementary to a region upstream and/or downstream of one or both of the nucleotides in position 108 and 171 of SEQ ID NO 5 or 1 and/or overlapping a region comprising one or both of the nucleotides in position 108 and 171 of SEQ ID NO 5 or 1.

9. The kit of claim 8 wherein one or more of said probes and/or oligonucleotides is labelled.

10. A nucleotide sequence of SEQ ID NO 1, said sequence coding for the rpl14 gene (381 bp) specific for the FD related phytoplasma.

## Patentansprüche

1. Verfahren für die Diagnose der Rebstockkrankheit Flavescence dorée, das den folgenden Schritt umfasst:
a) Identifizieren der Nukleotide in den Positionen 108 und/oder 171 von SEQ ID NO 5 und/oder ihrer komplementären Nukleotide in dem zu SEQ ID NO 5 komplementären Segment in einer Pflanzenprobe, die Phloem umfasst;
wobei die Anwesenheit eines C-Rückstands in Position 108 von SEQ ID NO 5 und/oder eines G-Rückstands in ihrem komplementären Nukleotid in der zu SEQ ID NO 5 komplementären Sequenz und/oder eines G-Rückstands in Position 171 vom SEQ ID NO 5 und/oder eines C-Rückstands in ihrem komplementären Nukleotid die Anwesenheit mindestens eines der Phytoplasma-Stämme, die mit der Rebstockkrankheit Flavescence dorée verknüpft sind, in der Probe anzeigt.

2. Verfahren gemäß Anspruch 1, wobei die Identifikation von Schritt a) umfasst: einen ersten Schritt, wobei eine PCR-, Real-Time-PCR-, Ligations-, allelspezifische Hybridisierungs-, Primer Extension-, invasive Spaltungs- oder Sequencing-Reaktion ausgeführt wird, und einen zweiten Schritt, wobei das in dem ersten Schritt erhaltene Produkt durch Überwachen des durch das Produkt emittierten Lichts oder Messen der Masse des Produkts oder Überwachen der durch das Produkt emittierten Radioaktivität oder durch Sequenzieren des Produkts detektiert wird.

3. Verfahren gemäß einem der Ansprüche 1 oder 2, wobei das Überwachen der Lichtemission durch Überwachen der Fluoreszenz, der Lumineszenz, der zeitlich aufgelösten Fluoreszenz, des Fluoreszenz-Resonanz-Energie-Transfers, oder der Fluoreszenz-Polarisation durchgeführt wird, wobei das Messen der Masse durch Massenspektrometrie oder Heteroduplexanalyse ausgeführt wird und das Überwachen der Radioaktivität mit radioaktivitätsempfindlichen Tools ausgeführt wird.

4. Verfahren gemäß einem der Ansprüche 1 - 3, wobei das Verfahren auf einem Festphasenformat, wie zum Beispiel einem Mikroarray oder einer Mikroplatte, durchgeführt wird.

5. Verfahren gemäß einem der Ansprüche 1 - 3, wobei die Identifizierung mit einer RT PCR unter Verwendung eines Vorwärts-Primers von SEQ ID NO 2, einer allelspezifischen Sonde von SEQ ID NO 3 und eines Rückwärts-Primers von SEQ ID NO 4 ausgeführt wird.

6. Kit für die Diagnose der Rebstockkrankheit Flavescence dorée, das umfasst: Reagenzien zur Identifizierung der Nukleotide in den Positionen 108 und 171 von SEQ ID NO 5 oder 1 und/oder ihrer komplementären Nukleotide in der zu SEQ ID NO 5 oder 1 komplementären Sequenz in einem Weinpflanzenphloem.

7. Kit gemäß Anspruch 6, das weiterhin feste Träger umfasst, wobei Oligonukleotide für die Identifizierung der Nukleotide in Position 108 und/oder 171 von SEQ ID NO 5 oder 1 und/oder ein oder beide ihrer komplementären Nukleotide in der zu SEQ ID NOR 5 oder 1 komplementären Sequenz in bekannten Positionen auf dem Träger in einer oder mehreren Kopien pro Position verankert sind.

8. Kit gemäß Anspruch 7, das weiterhin umfasst: gekennzeichnete Sonden und/oder Nukleotide, die einem Bereich entsprechen und/oder zu einem Bereich komplementär sind, der den Nukleotiden in den Positionen 108 und/oder 171 von SEQ ID NO 5 oder 1 vor- und/oder nachgelagert ist, und/oder einen Bereich überlappen, der die Nukleotide in den Positionen 108 und/oder 171 von SEQ ID NO 5 oder 1 umfasst.

9. Kit gemäß Anspruch 8, wobei eine oder mehrere der Sonden und/oder die Oligonukleotide gekennzeichnet sind.

10. Nukleotidsequenz von SEQ ID NO 1, wobei die Sequenz für das rpl14 Gen (381 bp) codiert, das für das FD verursachende Phytoplasma spezifisch ist.

## Revendications

1. Méthode de diagnostic de la maladie de la flavescence dorée de la vigne comprenant l'étape suivante
a) identification du ou des deux nucléotides à la position 108 et 171 de SEQ ID No: 5, et/ou de leurs nucléotides complémentaires dans la séquence complémentaire de SEQ ID No: 5, dans un échantillon de la plante comprenant le phloème ;
dans laquelle la présence d'un résidu C à la position 108 de SEQ ID No: 5, et/ou d'un résidu G dans son nucléotide complémentaire dans la séquence complémentaire de SEQ ID No: 5 et/ou d'un résidu G à la position 171 de SEQ ID No: 5, et/ou d'un résidu C dans son nucléotide complémentaire, indique la présence d'au moins une des souches du phytoplasme associé à la maladie de la flavescence dorée de la vigne dans l'échantillon.

2. Méthode selon la revendication 1 dans laquelle l'identification de l'étape a) comprend une première étape dans laquelle une PCR, une PCR en temps réel, une ligature, une hybridation spécifique de l'allèle, une extension d'amorce, un clivage invasif ou une réaction de séquençage est mis en oeuvre, et une seconde étape dans laquelle le produit obtenu à la première étape est détecté par surveillance de la lumière émise par ledit produit, ou mesure de la masse desdits produits, ou surveillance de la radioactivité émise par ledit produit, ou par séquençage dudit produit.

3. Méthode selon l'une quelconque des revendications 1 ou 2 dans laquelle ladite surveillance d'émission de lumière est mise en oeuvre par surveillance de la fluorescence, de la luminescence, de la fluorescence résolue en temps, du transfert d'énergie de fluorescence par résonance, ou de la polarisation de la fluorescence, ladite mesure de la masse est mise en oeuvre par spectrométrie de masse ou analyse hétéroduplex, et ladite surveillance de la radioactivité est mise en oeuvre à l'aide d'outils sensibles à la radioactivité.

4. Méthode selon l'une quelconque des revendications 1 à 3 dans laquelle ladite méthode est mise en oeuvre sur un format en phase solide tel qu'une micropuce ou une microplaque.

5. Méthode selon l'une quelconque des revendications 1 à 3 dans laquelle ladite identification est mise en oeuvre à l'aide d'une PCR RT en utilisant une amorce sens de type SEQ ID No: 2, une sonde spécifique de l'allèle de type SEQ ID No: 3 et une amorce antisens de type SEQ ID No: 4.

6. Kit pour le diagnostic de la maladie de la flavescence dorée de la vigne comprenant des réactifs pour l'identification des nucléotides à la position 108 et 171 de SEQ ID No: 5 ou 1 et/ou de leurs nucléotides complémentaires dans la séquence complémentaire de SEQ ID No: 5 ou 1 dans un phloème de pied de vigne.

7. Kit selon la revendication 6 comprenant en outre des supports solides dans lesquels des oligonucléotides pour l'identification du ou des deux nucléotides à la position 108 et 171 de SEQ ID No: 5 ou 1 et/ou de son ou de leurs deux nucléotides complémentaires dans la séquence complémentaire de SEQ ID No: 5 ou 1 sont ancrés à des positions connues sur ledit support à raison d'une ou de plusieurs copies par position.

8. Kit selon la revendication 7 comprenant en outre les sondes et/ou les oligonucléotides marqués correspondant à et/ou complémentaires d'une région en amont et/ou en aval du ou des deux nucléotides à la position 108 et 171 de SEQ ID No: 5 ou 1 et/ou chevauchant une région comprenant le ou les deux nucléotides à la position 108 et 171 de SEQ ID No: 5 ou 1.

9. Kit selon la revendication 8 dans lequel une ou plusieurs desdites sondes et/ou desdits oligonucléotides sont marqués.

10. Séquence de nucléotides de SEQ ID No: 1, ladite séquence codant pour le gène rpl14 (381 pb) spécifique du phytoplasme lié à la FD.
